# EUROPEAN PATENT SPECIFICATION

(11) **EP 1 151 118 B1**
(45) Date of publication and mention of the grant of the patent: **09.04.2008**
(21) Application number: 00905982.5
(22) Date of filing: 04.02.2000
(51) Int. Cl.: C12N 15/79, C12N 15/85, C12P 21/00, G01N 33/569, A61K 39/002, A61K 38/17

(54) **RECOMBINANT EXPRESSION OF HETEROLOGOUS NUCLEIC ACIDS IN PROTOZOA**
REKOMBINANTE EXPRESSION VON HETEROLOGEN NUKLEINSÄUREN IN PROTOZOEN
EXPRESSION RECOMBINANTE D'ACIDES NUCLEIQUES HETEROLOGUES DANS UN PROTOZOAIRE

(30) Priority: 04.02.1999 US 118634 P; 02.03.1999 US 122372 P; 17.03.1999 US 124905 P; 27.04.1999 US 131121 P
(43) Date of publication of application: 07.11.2001
(73) Proprietor: THE UNIVERSITY OF GEORGIA RESEARCH FOUNDATION, INC., Athens, Georgia 30602-7411 (US)
(72) Inventor: GAERTIG, Jacek, Athens, GA 30605 (US); DICKERSON, Harry, W., Jr., Athens, GA 30605 (US); CLARK, Theodore, G., Ithaca, NY 14850 (US)
(74) Representative: Vossius & Partner
(86) International application number: PCT/US2000/002966
(87) International publication number: WO 2000/046381

(56) References cited:
- US-B1- 7 026 156
- GAERTIG J ET AL: "Electroporation-mediated replacement of a positively and negatively selectable ß-tubulin gene in Tetrahymena thermophila" PROCEEDINGS OF THE NATIONAL ACADEMY OF SCIENCES OF USA, vol. 91, May 1994 (1994-05), pages 4549-4553, XP002141863 WASHINGTON US
- CLARK TG ET AL: "Developmental expression of surface antigen genes in the parasitic ciliate Ichtyophthirius multifiliis" PROCEEDINGS OF THE NATIONAL ACADEMY OF SCIENCES OF USA, vol. 89, July 1992 (1992-07), pages 6363-6367, XP002098111 WASHINGTON US
- GAERTIG ET AL: "High frequency vector-mediated transformation and gene replacement in Tetrahymena" NUCLEIC ACIDS RESEARCH,GB,OXFORD UNIVERSITY PRESS, SURREY, vol. 22, no. 24, 1994, pages 5391-5398-8, XP002128557 ISSN: 0305-1048 cited in the application
- KAHN R W ET AL: "TRANSFORMATION OF TETRAHYMENA THERMOPHILA BY MICROINJECTION OF A FOREIGN GENE" PROCEEDINGS OF THE NATIONAL ACADEMY OF SCIENCES OF USA,US,NATIONAL ACADEMY OF SCIENCE. WASHINGTON, vol. 90, no. 20, 15 October 1993 (1993-10-15), pages 9295-9299, XP002044342 ISSN: 0027-8424 cited in the application
- GAERTIG J: "Suface display of a parasite antigen in the ciliate Tetrahymena thermophila" NATURE BIOTECHNOLOGY., vol. 17, May 1999 (1999-05), pages 462-465, XP002141865 NATURE PUBLISHING., US ISSN: 1087-0156

### SEQUENCE LISTING

<110> GAERTIG, Jacek
   DICKERSON Jr., Harry W.
   CLARK, Theodore G.
   THE UNIVERSITY OF GEORGIA RESEARCH FOUNDATION, INC
<120> RECOMBINANT EXPRESSION OF HETEROLOGOUS NUCLEIC ACIDS IN PROTOZOA
<130> 235.00100201
<140> PCT/USOO/02966
   <141> 2000-02-04
<150> 60/118,634
   <151> 1999-02-04
<150> 60/122,372
   <151> 1999-03-02
<150> 60/124,905
   <151> 1999-03-17
<150> 60/131,121
   <151> 1999-04-27
<160> 14
<170> PatentIn Ver. 2.0
<210> 1
   <211> 2268
   <212> DNA
   <213> Artificial Sequence
<220>
   <223> Description of Artificial Sequence: BTU::neol construct
<400> 1
   ggatctctct ctctatcgta ttttgcaata ataggtatta acttttatac tgattgttag 60
   tagatgcctt caaatttctt tttatttaaa ttcatatcta tatcttttaa aacactccac 120
   attttattgt tgctaactgt gctatgatct ttaagtcaat agctgctcat tttgttgaac 180
   tccacagaga cactaaattt gtttattttg atggatgctt taattaaagt tgctaatctt 240
   gcttgacatt tagccaacta ttggaagatc aaaatgtagc ttaaatctca aaaaatcatc 300
   ataatttact atcaaattat taagaaattc atataacacc actttattga ctttttattc 360
   atcttataga gtgatagtag agttgagcca aattgatact tgtttacgtt gtattatttt 420
   acgttgtatt attttgaaat tttaaaaaaa tgaaaatgag agaaaaattt atttaaattt 480
   gagcttagaa tctttaagga agatcaaaaa tgggctaact aaatgttaga gtacgaagac 540
   tgttcttgaa ataaagtgtc atcctttacg aatcaagttg ctactttaat gaataataga 600
   atttgaggta gagctaaaat gagagatata gtaatgctat tggattatat ttggtttgta 660
   ttgatggttt ttctttggta aatgaatgat ataaatgaag agtggcaata aaattaattg 720
   aaattgaatg aaaaaatgaa tagaaattaa agaagagtat aattttattt tttgaaattt 780
   ttaattttaa aatttttaat gcgtgtattt tatttgggtg atgaaaaggg taaataaagg 840
   gcgttgcact tttctcaata gtaatattta agaagagagg gatttagaag gaaaaatcat 900
   aatcaaataa aaaaaataaa aaagtttgaa aaaaaacctt caaaaaaaat aaataaaaac 960
   aaaatcactc ctttaagcaa ttaaaaaatg gcaagcttgg atggattgca cgcaggttct 1020
   ccggccgctt gggtggagag gctattcggc tatgactggg cacaacagac aatcggctgc 1080
   tctgatgccg ccgtgttccg gctgtcagcg caggggcgcc cggttctttt tgtcaagacc 1140
   gacctgtccg gtgccctgaa tgaactgcag gacgaggcag cgcggctatc gtggctggcc 1200
   acgacgggcg ttccttgcgc agctgtgctc gacgttgtca ctgaagcggg aagggactgg 1260
   ctgctattgg gcgaagtgcc ggggcaggat ctcctgtcat ctcaccttgc tcctgccgag 1320
   aaagtatcca tcatggctga tgcaatgcgg cggctgcata cgcttgatcc ggctacctgc 1380
   ccattcgacc accaagcgaa acatcgcatc gagcgagcac gtactcggat ggaagccggt 1440
   cttgtcgatc aggatgatct ggacgaagag catcaggggc tcgcgccagc cgaactgttc 1500
   gccaggctca aggcgcgcat gcccgacggc gaggatctcg tcgtgaccca tggcgatgcc 1560
   tgcttgccga atatcatggt ggaaaatggc cgcttttctg gattcatcga ctgtggccgg 1620
   ctgggtgtgg cggaccgcta tcaggacata gcgttggcta cccgtgatat tgctgaagag 1680
   cttggcggcg aatgggctga ccgcttcctc gtgctttacg gtatcgccgc tcccgattcg 1740
   cagcgcatcg ccttctatcg ccttcttgac gagttcttct gaggatccac cgaactgaat 1800
   cggtcagcta aaccaaccaa tcaacataat aaactttatt atttttactt aagcatctta 1860
   ctgttgttgt aatagtagag aaagaaatac ccaattaact tcattcacat aacattaata 1920
   tctataaaca tcttttttct cacatatata caactctcta aatcaacaaa taacttttta 1980
   aaaataatgg atatatatta acaaaataat atatctcttt ttacaaaata gttcttatat 2040
   aaatacgtat ctgcactcac ccgcattttt cacaacaaaa acataccaaa aaaattctta 2100
   cttctacatg tttccttctt attattcaaa attattttat aaatagcata aaataaatac 2160
   aataaaaaaa taaacaaaat ccttttttta ttttgaatta tttaaaacaa atattttcaa 2220
   tcaatcagtc agtcagcata atataaagca acaaaacaaa ccaagttg            2268
<210> 2
   <211> 2268
   <212> DNA
   <213> Artificial Sequence
<220>
   <223> Description of Artificial Sequence: Complement of BTU::neo 1 construct
<400> 2
   caacttggtt tgttttgttg ctttatatta tgctgactga ctgattgatt gaaaatattt 60
   gttttaaata attcaaaata aaaaaaggat tttgtttatt tttttattgt atttatttta 120
   tgctatttat aaaataattt tgaataataa gaaggaaaca tgtagaagta agaatttttt 180
   tggtatgttt ttgttgtgaa aaatgcgggt gagtgcagat acgtatttat ataagaacta 240
   ttttgtaaaa agagatatat tattttgtta atatatatcc attattttta aaaagttatt 300
   tgttgattta gagagttgta tatatgtgag aaaaaagatg tttatagata ttaatgttat 360
   gtgaatgaag ttaattgggt atttctttct ctactattac aacaacagta agatgcttaa 420
   gtaaaaataa taaagtttat tatgttgatt ggttggttta gctgaccgat tcagttcggt 480
   ggatcctcag aagaactcgt caagaaggcg atagaaggcg atgcgctgcg aatcgggagc 540
   ggcgataccg taaagcacga ggaagcggtc agcccattcg ccgccaagct cttcagcaat 600
   atcacgggta gccaacgcta tgtcctgata gcggtccgcc acacccagcc ggccacagtc 660
   gatgaatcca gaaaagcggc cattttccac catgatattc ggcaagcagg catcgccatg 720
   ggtcacgacg agatcctcgc cgtcgggcat gcgcgccttg agcctggcga acagttcggc 780
   tggcgcgagc ccctgatgct cttcgtccag atcatcctga tcgacaagac cggcttccat 840
   ccgagtacgt gctcgctcga tgcgatgttt cgcttggtgg tcgaatgggc aggtagccgg 900
   atcaagcgta tgcagccgcc gcattgcatc agccatgatg gatactttct cggcaggagc 960
   aaggtgagat gacaggagat cctgccccgg cacttcgccc aatagcagcc agtcccttcc 1020
   cgcttcagtg acaacgtcga gcacagctgc gcaaggaacg cccgtcgtgg ccagccacga 1080
   tagccgcgct gcctcgtcct gcagttcatt cagggcaccg gacaggtcgg tcttgacaaa 1140
   aagaaccggg cgcccctgcg ctgacagccg gaacacggcg gcatcagagc agccgattgt 1200
   ctgttgtgcc cagtcatagc cgaatagcct ctccacccaa gcggccggag aacctgcgtg 1260
   caatccatcc aagcttgcca ttttttaatt gcttaaagga gtgattttgt ttttatttat 1320
   tttttttgaa ggtttttttt caaacttttt tatttttttt atttgattat gatttttcct 1380
   tctaaatccc tctcttctta aatattacta ttgagaaaag tgcaacgccc tttatttacc 1440
   cttttcatca cccaaataaa atacacgcat taaaaatttt aaaattaaaa atttcaaaaa 1500
   ataaaattat actcttcttt aatttctatt cattttttca ttcaatttca attaatttta 1560
   ttgccactct tcatttatat cattcattta ccaaagaaaa accatcaata caaaccaaat 1620
   ataatccaat agcattacta tatctctcat tttagctcta cctcaaattc tattattcat 1680
   taaagtagca acttgattcg taaaggatga cactttattt caagaacagt cttcgtactc 1740
   taacatttag ttagcccatt tttgatcttc cttaaagatt ctaagctcaa atttaaataa 1800
   atttttctct cattttcatt tttttaaaat ttcaaaataa tacaacgtaa aataatacaa 1860
   cgtaaacaag tatcaatttg gctcaactct actatcactc tataagatga ataaaaagtc 1920
   aataaagtgg tgttatatga atttcttaat aatttgatag taaattatga tgattttttg 1980
   agatttaagc tacattttga tcttccaata gttggctaaa tgtcaagcaa gattagcaac 2040
   tttaattaaa gcatccatca aaataaacaa atttagtgtc tctgtggagt tcaacaaaat 2100
   gagcagctat tgacttaaag atcatagcac agttagcaac aataaaatgt ggagtgtttt 2160
   aaaagatata gatatgaatt taaataaaaa gaaatttgaa ggcatctact aacaatcagt 2220
   ataaaagtta atacctatta ttgcaaaata cgatagagag agagatcc 2268
<210> 3
   <211> 2811
   <212> DNA
   <213> Artificial Sequence
<220>
   <223> Description of Artificial Sequence: Complement of BTU::neo 1 construct
<400> 3
   ggatctctct ctctatcgta ttttgcaata ataggtatta acttttatac tgattgttag 60
   tagatgcctt caaatttctt tttatttaaa ttcatatcta tatcttttaa aacactccac 120
   attttattgt tgctaactgt gctatgatct ttaagtcaat agctgctcat tttgttgaac 180
   tccacagaga cactaaattt gtttattttg atggatgctt taattaaagt tgctaatctt 240
   gcttgacatt tagccaacta ttggaagatc aaaatgtagc ttaaatctca aaaaatcatc 300
   ataatttact atcaaattat taagaaattc atataacacc actttattga ctttttattc 360
   atcttataga gtgatagtag agttgagcca aattgatact tgtttacgtt gtattatttt 420
   acgttgtatt attttgaaat tttaaaaaaa tgaaaatgag agaaaaattt atttaaattt 480
   gagcttagaa tctttaagga agatcaaaaa tgggctaact aaatgttaga gtacgaagac 540
   tgttcttgaa ataaagtgtc atcctttacg aatcaagttg ctactttaat gaataataga 600
   atttgaggta gagctaaaat gagagatata gtaatgctat tggattatat ttggtttgta 660
   ttgatggttt ttctttggta aatgaatgat ataaatgaag agtggcaata aaattaattg 720
   aaattgaatg aaaaaatgaa tagaaattaa agaagagtat aattttattt tttgaaattt 780
   ttaattttaa aatttttaat gcgtgtattt tatttgggtg atgaaaaggg taaataaagg 840
   gcgttgcact tttctcaata gtaatattta agaagagagg gatttagaag gaaaaatcat 900
   aatcaaataa aaaaaataaa aaagtttgaa aaaaaacctt caaaaaaaat aaataaaaac 960
   aaaatcactc ctttaagcaa ttaaaaaatg gcaagcttga aatataatat tttattaatt 1020
   ttaattattt ctttatttat taatgaatta agagctgttc catgtcctga tggtacttag 1080
   actcaagctg gattgactga tgtaggtgct gctgatcttg gtacttgtgt taattgcaga 1140
   cctaattttt actataatgg tggtgctgct taaggagaag ctaatggtaa ttaacctttc 1200
   gcagcaaata atgctgctag aggtatatgt gtaccatgcc aaataaacag agtaggctct 1260
   gttaccaatg caggtgactt agctacttta gccacataat gcagtactta atgtcctact 1320
   ggcactgcac ttgatgatgg agtgacagat gtttttgata gatcagccgc ataatgtgtt 1380
   aaatgcaaac ctaactttta ctataatggt ggttctcctt aaggtgaagc tcctggcgtt 1440
   taagtttttg ctgctggtgc tgccgctgca ggtgttgctg ccgttactag ttaatgtgta 1500
   ccttgccaac taaacaaaaa cgattctcct gccactgcag gtgcctaagc taatttagcc 1560
   acataatgta gcaattaatg tcctactggc actgtacttg atgatggagt gacacttgtt 1620
   tttaatacat cagccacatt atgtgttaaa tgcagaccta acttttacta taatggtggt 1680
   tctccttaag gtgaagctcc tggcgtttaa gtttttgctg ctggtgctgc cgctgcaggt 1740
   gttgctgccg ttactagtta atgtgtacct tgccaaataa acaaaaacga ttctcctgcc 1800
   tctgcaggtg cctaagctaa tttagccaca taatgcagta cttaatgtcc aactggcact 1860
   gcaattcaag acggagtgac acttgttttt agtaattcat ccacataatg ttcttaatgc 1920
   attgctaatt acttttttaa tggtaatttc gaagcaggta aaagttaatg tttaaagtgt 1980
   ccagtaagta aaactactcc agcacatgct ccaggtaata ctgctactta agccacataa 2040
   tgtttgacca catgtcctgc tggtacagta cttgatgatg gaacatcaac taattttgta 2100
   gcttccgcaa ctgaatgtac taaatgttct gctggctttt ttgcatcaaa aacaactggt 2160
   tttacagcag gtactgatac atgtactgaa tgtactaaaa aattaacttc tggtgccaca 2220
   gctaaagtat atgctgaagc tactcaaaaa gtataatgcg cctccactac tttcgctaaa 2280
   tttttatcga tttccttatt atttatttct ttctatttat tgtgaggatc caccgaactg 2340
   aatcggtcag ctaaaccaac caatcaacat aataaacttt attattttta cttaagcatc 2400
   ttactgttgt tgtaatagta gagaaagaaa tacccaatta acttcattca cataacatta 2460
   atatctataa acatcttttt tctcacatat atacaactct ctaaatcaac aaataacttt 2520
   ttaaaaataa tggatatata ttaacaaaat aatatatctc tttttacaaa atagttctta 2580
   tataaatacg tatctgcact cacccgcatt tttcacaaca aaaacatacc aaaaaaattc 2640
   ttacttctac atgtttcctt cttattattc aaaattattt tataaatagc ataaaataaa 2700
   tacaataaaa aaataaacaa aatccttttt ttattttgaa ttatttaaaa caaatatttt 2760
   caatcaatca gtcagtcagc ataatataaa gcaacaaaac aaaccaagtt g          2811
<210> 4
   <211> 2811
   <212> DNA
   <213> Artificial Sequence
<220>
   <223> Description of Artificial Sequence: Complement of pBICH 3 vector construct
<400> 4
   caacttggtt tgttttgttg ctttatatta tgctgactga ctgattgatt gaaaatattt 60
   gttttaaata attcaaaata aaaaaaggat tttgtttatt tttttattgt atttatttta 120
   tgctatttat aaaataattt tgaataataa gaaggaaaca tgtagaagta agaatttttt 180
   tggtatgttt ttgttgtgaa aaatgcgggt gagtgcagat acgtatttat ataagaacta 240
   ttttgtaaaa agagatatat tattttgtta atatatatcc attattttta aaaagttatt 300
   tgttgattta gagagttgta tatatgtgag aaaaaagatg tttatagata ttaatgttat 360
   gtgaatgaag ttaattgggt atttctttct ctactattac aacaacagta agatgcttaa 420
   gtaaaaataa taaagtttat tatgttgatt ggttggttta gctgaccgat tcagttcggt 480
   ggatcctcac aataaataga aagaaataaa taataaggaa atcgataaaa atttagcgaa 540
   agtagtggag gcgcattata ctttttgagt agcttcagca tatactttag ctgtggcacc 600
   agaagttaat tttttagtac attcagtaca tgtatcagta cctgctgtaa aaccagttgt 660
   ttttgatgca aaaaagccag cagaacattt agtacattca gttgcggaag ctacaaaatt 720
   agttgatgtt ccatcatcaa gtactgtacc agcaggacat gtggtcaaac attatgtggc 780
   ttaagtagca gtattacctg gagcatgtgc tggagtagtt ttacttactg gacactttaa 840
   acattaactt ttacctgctt cgaaattacc attaaaaaag taattagcaa tgcattaaga 900
   acattatgtg gatgaattac taaaaacaag tgtcactccg tcttgaattg cagtgccagt 960
   tggacattaa gtactgcatt atgtggctaa attagcttag gcacctgcag aggcaggaga 1020
   atcgtttttg tttatttggc aaggtacaca ttaactagta acggcagcaa cacctgcagc 1080
   ggcagcacca gcagcaaaaa cttaaacgcc aggagcttca ccttaaggag aaccaccatt 1140
   atagtaaaag ttaggtctgc atttaacaca taatgtggct gatgtattaa aaacaagtgt 1200
   cactccatca tcaagtacag tgccagtagg acattaattg ctacattatg tggctaaatt 1260
   agcttaggca cctgcagtgg caggagaatc gtttttgttt agttggcaag gtacacatta 1320
   actagtaacg gcagcaacac ctgcagcggc agcaccagca gcaaaaactt aaacgccagg 1380
   agcttcacct taaggagaac caccattata gtaaaagtta ggtttgcatt taacacatta 1440
   tgcggctgat ctatcaaaaa catctgtcac tccatcatca agtgcagtgc cagtaggaca 1500
   ttaagtactg cattatgtgg ctaaagtagc taagtcacct gcattggtaa cagagcctac 1560
   tctgtttatt tggcatggta cacatatacc tctagcagca ttatttgctg cgaaaggtta 1620
   attaccatta gcttctcctt aagcagcacc accattatag taaaaattag gtctgcaatt 1680
   aacacaagta ccaagatcag cagcacctac atcagtcaat ccagcttgag tctaagtacc 1740
   atcaggacat ggaacagctc ttaattcatt aataaataaa gaaataatta aaattaataa 1800
   aatattatat ttcaagcttg ccatttttta attgcttaaa ggagtgattt tgtttttatt 1860
   tatttttttt gaaggttttt tttcaaactt ttttattttt tttatttgat tatgattttt 1920
   ccttctaaat ccctctcttc ttaaatatta ctattgagaa aagtgcaacg ccctttattt 1980
   acccttttca tcacccaaat aaaatacacg cattaaaaat tttaaaatta aaaatttcaa 2040
   aaaataaaat tatactcttc tttaatttct attcattttt tcattcaatt tcaattaatt 2100
   ttattgccac tcttcattta tatcattcat ttaccaaaga aaaaccatca atacaaacca 2160
   aatataatcc aatagcatta ctatatctct cattttagct ctacctcaaa ttctattatt 2220
   cattaaagta gcaacttgat tcgtaaagga tgacacttta tttcaagaac agtcttcgta 2280
   ctctaacatt tagttagccc atttttgatc ttccttaaag attctaagct caaatttaaa 2340
   taaatttttc tctcattttc atttttttaa aatttcaaaa taatacaacg taaaataata 2400
   caacgtaaac aagtatcaat ttggctcaac tctactatca ctctataaga tgaataaaaa 2460
   gtcaataaag tggtgttata tgaatttctt aataatttga tagtaaatta tgatgatttt 2520
   ttgagattta agctacattt tgatcttcca atagttggct aaatgtcaag caagattagc 2580
   aactttaatt aaagcatcca tcaaaataaa caaatttagt gtctctgtgg agttcaacaa 2640
   aatgagcagc tattgactta aagatcatag cacagttagc aacaataaaa tgtggagtgt 2700
   tttaaaagat atagatatga atttaaataa aaagaaattt gaaggcatct actaacaatc 2760
   agtataaaag ttaataccta ttattgcaaa atacgataga gagagagatc c 2811
<210> 5
   <211> 442
   <212> PRT
   <213> Ichthyophthirius multifiliis
<400> 5
   Met Lys Tyr Asn Ile Leu Leu Ile Leu Ile Ile Ser Leu Phe Ile Asn
   1               5                   10                  15
   Glu Leu Arg Ala Val Pro Cys Pro Asp Gly Thr Gln Thr Gln Ala Gly
               20                  25                  30
   Leu Thr Asp Val Gly Ala Ala Asp Leu Gly Thr Cys Val Asn Cys Arg
           35                  40                  45
   Pro Asn Phe Tyr Tyr Asn Gly Gly Ala Ala Gln Gly Glu Ala Asn Gly
       50                  55                  60
   Asn Gln Pro Phe Ala Ala Asn Asn Ala Ala Arg Gly Ile Cys Val Pro
   65                  70                  75                  80
   Cys Gln Ile Asn Arg Val Gly Ser Val Thr Asn Ala Gly Asp Leu Ala
                   85                  90                  95
   Thr Leu Ala Thr Gln Cys Ser Thr Gln Cys Pro Thr Gly Thr Ala Leu
               100                 105                 110
   Asp Asp Gly Val Thr Asp Val Phe Asp Arg Ser Ala Ala Gln Cys Val
           115                 120                 125
   Lys Cys Lys Pro Asn Phe Tyr Tyr Asn Gly Gly Ser Pro Gln Gly Glu
       130                 135                 140
   Ala Pro Gly Val Gln Val Phe Ala Ala Gly Ala Ala Ala Ala Gly Val
   145                 150                 155                 160
   Ala Ala Val Thr Ser Gln Cys Val Pro Cys Gln Leu Asn Lys Asn Asp
                   165                 170                 175
   Ser Pro Ala Thr Ala Gly Ala Gln Ala Asn Leu Ala Thr Gln Cys Ser
               180                 185                 190
   Asn Gln Cys Pro Thr Gly Thr Val Leu Asp Asp Gly Val Thr Leu Val
           195                 200                 205
   Phe Asn Thr Ser Ala Thr Leu Cys Val Lys Cys Arg Pro Asn Phe Tyr
       210                 215                 220
   Tyr Asn Gly Gly Ser Pro Gln Gly Glu Ala Pro Gly Val Gln Val Phe
   225                 230                 235                 240
   Ala Ala Gly Ala Ala Ala Ala Gly Val Ala Ala Val Thr Ser Gln Cys
                   245                 250                 255
   Val Pro Cys Gln Ile Asn Lys Asn Asp Ser Pro Ala Thr Ala Gly Ala
               260                 265                 270
   Gln Ala Asn Leu Ala Thr Gln Cys Ser Thr Gln Cys Pro Thr Gly Thr
           275                 280                 285
   Ala Ile Gln Asp Gly Val Thr Leu Val Phe Ser Asn Ser Ser Thr Gln
       290                 295                 300
   Cys Ser Gln Cys Ile Ala Asn Tyr Phe Phe Asn Gly Asn Phe Glu Ala
   305                 310                 315                 320
   Gly Lys Ser Gln Cys Leu Lys Cys Pro Val Ser Lys Thr Thr Pro Ala
                   325                 330                 335
   His Ala Pro Gly Asn Thr Ala Thr Gln Ala Thr Gln Cys Leu Thr Thr
               340                 345                 350
   Cys Pro Ala Gly Thr Val Leu Asp Asp Gly Thr Ser Thr Asn Phe Val
           355                 360                 365
   Ala Ser Ala Thr Glu Cys Thr Lys Cys Ser Ala Gly Phe Phe Ala Ser
       370                 375                 380
   Lys Thr Thr Gly Phe Thr Ala Gly Thr Asp Thr Cys Thr Glu Cys Thr
   385                 390                 395                 400
   Lys Lys Leu Thr Ser Gly Ala Thr Ala Lys Val Tyr Ala Glu Ala Thr
                   405                 410                 415
   Gln Lys Val Gln Cys Ala Ser Thr Thr Phe Ala Lys Phe Leu Ser Ile
               420                 425                 430
   Ser Leu Leu Phe Ile Ser Phe Tyr Leu Leu
           435                 440
<210> 6
   <211> 468
   <212> PRT
   <213> Ichthyophthirius multifiliis
<400> 6
   Met Lys Asn Asn Ile Leu Val Ile Leu Ile Ile Ser Leu Phe Ile Asn
   1               5                   10                  15
   Gln Ile Lys Ser Ala Asn Cys Pro Val Gly Thr Glu Thr Asn Thr Ala
               20                  25                  30
   Gly Gln Val Asp Asp Leu Gly Thr Pro Ala Asn Cys Val Asn Cys Gln
           35                  40                  45
   Lys Asn Phe Tyr Tyr Asn Asn Ala Ala Ala Phe Val Pro Gly Ala Ser
       50                  55                  60
   Thr Cys Thr Pro Cys Pro Gln Lys Lys Asp Ala Gly Ala Gln Pro Asn
   65                  70                  75                  80
   Pro Pro Ala Thr Ala Asn Leu Val Thr Gln Cys Asn Val Lys Cys Pro
                   85                  90                  95
   Ala Gly Thr Ala Ile Ala Gly Gly Ala Thr Asp Tyr Ala Ala Ile Ile
               100                 105                 110
   Thr Glu Cys Val Asn Cys Arg Ile Asn Phe Tyr Asn Glu Asn Ala Pro
           115                 120                 125
   Asn Phe Asn Ala Gly Ala Ser Thr Cys Thr Ala Cys Pro Val Asn Arg
       130                 135                 140
   Val Gly Gly Ala Leu Thr Ala Gly Asn Ala Ala Thr Ile Val Ala Gln
   145                 150                 155                 160
   Cys Asn Val Ala Cys Pro Thr Gly Thr Ala Leu Asp Asp Gly Val Thr
                   165                 170                 175
   Thr Asp Tyr Val Arg Ser Phe Thr Glu Cys Val Lys Cys Arg Leu Asn
               180                 185                 190
   Phe Tyr Tyr Asn Gly Asn Asn Gly Asn Thr Pro Phe Asn Pro Gly Lys
           195                 200                 205
   Ser Gln Cys Thr Pro Cys Pro Ala Ile Lys Pro Ala Asn Val Ala Gln
       210                 215                 220
   Ala Thr Leu Gly Asn Asp Ala Thr Ile Thr Ala Gln Cys Asn Val Ala
   225 230 235 240
   Cys Pro Asp Gly Thr Ile Ser Ala Ala Gly Val Asn Asn Trp Val Ala
   245 250 255
   Gln Asn Thr Glu Cys Thr Asn Cys Ala Pro Asn Phe Tyr Asn Asn Asn
   260 265 270
   Ala Pro Asn Phe Asn Pro Gly Asn Ser Thr Cys Leu Pro Cys Pro Ala
   275 280 285
   Asn Lys Asp Tyr Gly Ala Glu Ala Thr Ala Gly Gly Ala Ala Thr Leu
   290 295 300
   Ala Lys Gln Cys Asn Ile Ala Cys Pro Asp Gly Thr Ala Ile Ala Ser
   305 310 315 320
   Gly Ala Thr Asn Tyr Val Ile Leu Gln Thr Glu Cys Leu Asn Cys Ala
   325 330 335
   Ala Asn Phe Tyr Phe Asp Gly Asn Asn Phe Gln Ala Gly Ser Ser Arg
   340 345 350
   Cys Lys Ala Cys Pro Ala Asn Lys Val Gln Gly Ala Val Ala Thr Ala
   355 360 365
   Gly Gly Thr Ala Thr Leu Ile Ala Gln Cys Ala Leu Glu Cys Pro Ala
   370 375 380
   Gly Thr Val Leu Thr Asp Gly Thr Thr Ser Thr Tyr Lys Gln Ala Ala
   385 390 395 400
   Ser Glu Cys Val Lys Cys Ala Ala Asn Phe Tyr Thr Thr Lys Gln Thr
   405 410 415
   Asp Trp Val Ala Gly Ile Asp Thr Cys Thr Ser Cys Asn Lys Lys Leu
   420 425 430
   Thr Ser Gly Ala Glu Ala Asn Leu Pro Glu Ser Ala Lys Lys Asn Ile
   435 440 445
   Gln Cys Asp Phe Ala Asn Phe Leu Ser Ile Ser Leu Leu Leu Ile Ser
   450 455 460
   Tyr Tyr Leu Leu
   465
<210> 7
   <211> 1326
   <212> DNA
   <213> Ichthyophthirius multifiliis
<400> 7
   atgaaatata atattttatt aattttaatt atttctttat ttattaatga attaagagct 60
   gttccatgtc ctgatggtac ttagactcaa gctggattga ctgatgtagg tgctgctgat 120
   cttggtactt gtgttaattg cagacctaat ttttactata atggtggtgc tgcttaagga 180
   gaagctaatg gtaattaacc tttcgcagca aataatgctg ctagaggtat atgtgtacca 240
   tgccaaataa acagagtagg ctctgttacc aatgcaggtg acttagctac tttagccaca 300
   taatgcagta cttaatgtcc tactggcact gcacttgatg atggagtgac agatgttttt 360
   gatagatcag ccgcataatg tgttaaatgc aaacctaact tttactataa tggtggttct 420
   ccttaaggtg aagctcctgg cgtttaagtt tttgctgctg gtgctgccgc tgcaggtgtt 480
   gctgccgtta ctagttaatg tgtaccttgc caactaaaca aaaacgattc tcctgccact 540
   gcaggtgcct aagctaattt agccacataa tgtagcaatt aatgtcctac tggcactgta 600
   cttgatgatg gagtgacact tgtttttaat acatcagcca cattatgtgt taaatgcaga 660
   cctaactttt actataatgg tggttctcct taaggtgaag ctcctggcgt ttaagttttt 720
   gctgctggtg ctgccgctgc aggtgttgct gccgttacta gttaatgtgt accttgccaa 780
   ataaacaaaa acgattctcc tgccactgca ggtgcctaag ctaatttagc cacataatgc 840
   agtacttaat gtccaactgg cactgcaatt caagacggag tgacacttgt ttttagtaat 900
   tcatccacat aatgttctta atgcattgct aattactttt ttaatggtaa tttcgaagca 960
   ggtaaaagtt aatgtttaaa gtgtccagta agtaaaacta ctccagcaca tgctccaggt 1020
   aatactgcta cttaagccac ataatgtttg accacatgtc ctgctggtac agtacttgat 1080
   gatggaacat caactaattt tgtagcttcc gcaactgaat gtactaaatg ttctgctggc 1140
   ttttttgcat caaaaacaac tggttttaca gcaggtactg atacatgtac tgaatgtact 1200
   aaaaaattaa cttctggtgc cacagctaaa gtatatgctg aagctactca aaaagtataa 1260
   tgcgcctcca ctactttcgc taaattttta tcgatttcct tattatttat ttctttctat 1320
   ttattg 1326
<210> 8
   <211> 1404
   <212> DNA
   <213> Ichthyophthirius multifiliis
<400> 8
   atgaaaaata atattttagt aatattgatt atttcattat ttatcaatta aattaaatct 60
   gctaattgtc ctgttggaac tgaaactaac acagccggat aagttgatga tctaggaact 120
   cctgcaaatt gtgttaattg ttagaaaaac ttttattata ataatgctgc tgctttcgtt 180
   cctggtgcta gtacgtgtac accttgtcca taaaaaaaag atgctggtgc ttaaccaaat 240
   ccacctgcta ctgctaattt agtcacataa tgtaacgtta aatgccctgc tggtaccgca 300
   attgcaggtg gagcaacaga ttatgcagca ataatcacag aatgtgttaa ttgtagaatt 360
   aatttttata atgaaaatgc tccaaatttt aatgcaggtg ctagtacatg cacagcttgt 420
   ccggtaaaca gagttggtgg tgcattgact gctggtaatg ccgctaccat agtcgcataa 480
   tgtaacgtcg catgtcctac tggtactgca cttgatgatg gagtaactac tgattatgtt 540
   agatcattca cagaatgtgt taaatgtaga cttaactttt actataatgg taataatggt 600
   aatactcctt tcaatccagg taaaagttaa tgcacacctt gtccggcaat taaacctgct 660
   aatgttgctt aagctacttt aggtaatgat gctacaataa ccgcataatg taacgttgca 720
   tgccctgatg gtactataag tgctgctgga gtaaataatt gggtagcaca aaacactgaa 780
   tgtactaatt gtgctcctaa cttttacaat aataatgctc ctaatttcaa tccaggtaat 840
   agtacatgcc taccttgccc agcaaataaa gattatggtg ctgaagccac tgcaggtggt 900
   gccgctactt tagccaaata atgtaatatt gcatgccctg atggtactgc aattgctagt 960
   ggagcaacta attatgtaat attataaaca gaatgtctaa attgtgctgc taacttttat 1020
   tttgatggta ataatttcta ggcaggaagt agtagatgca aagcatgtcc agcaaataaa 1080
   gtttaaggcg ctgtagcaac tgcaggtggt actgctactt taattgcata atgtgccctt 1140
   gaatgccctg ctggtactgt actcaccgat ggaacaacat ctacttataa ataagcagca 1200
   tctgaatgtg ttaaatgtgc tgccaacttt tatactacaa aataaactga ttgggtagca 1260
   ggtattgata catgtactag ttgtaataaa aaattaactt ctggcgctga agctaattta 1320
   cctgaatctg ctaaaaaaaa tatataatgt gatttcgcta attttttatc aatttcctta 1380
   ttattgattt cttattattt atta                                       1404
<210> 9
   <211> 28
   <212> DNA
   <213> Artificial Sequence
<220>
   <223> Description of Artificial Sequence: primer
<400> 9
   agccagtccc ttcccgcttc agtgacaa 28
<210> 10
   <211> 29
   <212> DNA
   <213> Artificial Sequence
<220>
   <223> Description of Artificial Sequence: primer
<400> 10
   cgggatccag cgaactgaat cggtcagct 29
<210> 11
   <211> 36
   <212> DNA
   <213> Artificial Sequence
<220>
   <223> Description of Artificial Sequence: primer
<400> 11
   cccagcttga aatataatat tttattaatt ttaatt 36
<210> 12
   <211> 31
   <212> DNA
   <213> Artificial Sequence
<220>
   <223> Description of Artificial Sequence: primer
<400> 12
   agggatcctc acaataaata gaaagaaata a 31
<210> 13
   <211> 23
   <212> DNA
   <213> Artificial Sequence
<220>
   <223> Description of Artificial Sequence: primer
<400> 13
   aaaaaataaa agtttgaaaa aaa 23
<210> 14
   <211> 23
   <212> DNA
   <213> Artificial Sequence
<220>
   <223> Description of Artificial Sequence: primer
<400> 14
   gtttagctga ccgattcagt tcg 23

## Description

### Statement of Government Rights

This invention was made with government support under grants from the National Institutes of Health (GM-54017-03) and the United States Department of Agriculture National Research Initiative Competitive Grants Program (NRICGP) (95-37204-2139). The U.S. government has certain rights in this invention.

### Field of the Invention

The present invention relates to the field of recombinant protein production, particularly recombinant protein production in nonpathogenic protozoa, such as the ciliate *Tetrahymena.*

### Background of the Invention

Efficient and high-level recombinant production of heterologous proteins is an important alternative to chemical synthesis and the isolation of proteins from native sources. Recombinant protein production is especially useful when the native protein is normally produced in limited amounts or by sources which are impossible, expensive and/or dangerous to obtain or propagate. Although a number of recombinant expression systems have proven useful for production of various heterologous proteins, none of these systems is universally applicable for the production of all proteins. For instance, *E. coli* appears to lack the ability to provide many post-translational modifications to heterologous proteins. Yeast can provide only some post-translational modifications (e.g., glycosylation patterns), and rapid degradation of heterologous proteins in yeast is common. Additionally, heterologous proteins secreted by yeast may contain long, untrimmed oligosaccharide chains, which sometimes results in biologically inactive or antigenically altered proteins. Moreover, a replacement of the natural mammalian signal peptide with a yeast signal peptide is almost always required for efficient secretion of mammalian proteins by yeast. Expression of heterologous eukaryotic proteins in insect or mammalian cells can be more reliable but both require expensive media for cell propagation. Moreover, cultured insect cells and mammalian cells have a relatively long doubling time compared to conventional bacterial systems such as *E. coli* and certain protozoa such as *Tetrahymena.*

Protozoa represent an alternative for the recombinant production of heterologous proteins, however few protozoa have been characterized to the extent necessary for routine heterologous protein expression. Well-characterized pathogenic protozoa that have been genetically engineered to express heterologous proteins include *Trypanosoma cruzi, Trypanosoma brucei,* and *Leishmania* spp. A number of shuttle vectors designed for episomal replication and coding region expression in pathogenic protozoa have been developed. An inducible coding region expression system has been established for pathogenic *T. brucei* (Wirtz, E., et al., Science, 268, 1179-1183 (1995)). Vectors that allow efficient coding region expression in different hosts like *E. coli* and mammalian cells have also been developed (Al-Qahtani, A., et al., Nucleic Acids Res., 24, 1173-1174 (1996)).

Protozoa are characterized by a glycosylphosphatidylinositol (GPI) anchoring system that allows targeted surface expression, or "display," of various endogenous proteins. Recent experiments in the kinetoplastid *Trypanosoma cruzi* demonstrated that mammalian and protozoan signal peptides function in *T. cruzi* to target a heterologous protein to different cellular compartments, and further showed both secretion and GPI-anchored surface expression in *T. cruzi* of a heterologous protein (Garg et al., J. Immunol., 158: 3293-3302 (1997)). Surface display in *T. cruzi* of chicken ovalbumin (OVA) was achieved using a construct comprising the signal sequence of *T. cruzi* glycoprotein, gp-72, that targets the protein to the endoplasmic reticulum, followed by a coding region for OVA, followed by 45 amino acids of amastigote surface protein I of *T. cruzi* which provided a C-terminal hydrophobic tail containing GPI anchor cleavage/attachment site. The protein thus anchored to the surface of the protozoan via a GPI structure was found to be readily presented in association with class I MHC by parasite-infected host cells.

Heterologous proteins have also been expressed in the slime mold *Dictyostelium discoideum.* A number of proteins have been expressed in this system including surface expression of the malaria circumsporozoite antigen (CSP) (Reymond et al., J. Biol. Chem. 1995, 270: 12941-12947); see Williams et al., Current Biology, 1995, 6:538-542, for a review).

Bioactive cytokines (IL-2 and IFN-γ) have also been produced in both *T. cruzi* and *Leishmania* (La Flamme et al., Mol. Biochem. Parasitol., 75:25-31 (1995), and Tobin et al., J. Immunol., 150:5059-5069 (1995)) in experiments that suggest that mammalian signal peptides are recognized and processed by these protozoa. However, pathogenic protozoa have not been exploited as a general purpose protein expression system, presumably because they are difficult or expensive to grow in large numbers and/or are infectious to human beings.

The nonpathogenic ciliate protozoan *Tetrahymena* has also been explored as a vehicle for expression of heterologous genes, but with limited success to date. *T. thermophila* has been successfully transformed using self-replicating palindromic ribosomal DNA (rDNA) purified from macronuclei (Tondravi et al., Proc. Natl. Acad. Sci. USA 83:4369 (1986)). Selection of transformants relied on a dominant paromomycin-resistance mutation in the 17S rRNA. rDNA-based shuttle vectors capable of autonomously replicating in *Tetrahymena* as well as in *E. coli* have also been developed; these plasmids contained a replication origin (ori) from the *T. thermophila* rDNA minichromosome (Yu et al., Proc. Natl. Acad. Sci. USA 86:8487-8491 (1989)).

rDNA vectors are usually circular vectors containing both regulatory regions and "coding" regions for *Tetrahymena* rRNA. A typical somatic rDNA vector contains a 5' nontranscribed sequence (5'-NTS), followed by a "coding" region for rRNA, followed by a 3' nontranscribed sequence (3'-NTS). A transgene is inserted into the 3' NTS. Somatic rDNA vectors contain the macronuclear version of rDNA and transform either by replacement of the macronuclear rDNA gene via homologous recombination or by autonomous replication as an extrachromosomal element. Processing rDNA vectors, on the other hand, contain additional processing signals upstream and downstream from the 5'-NTS and the 3'-NTS, respectively, obtained from the micronuclear version of rDNA. Processing rDNA vectors mimic what happens to the micronucleus rDNA in the newly developing macronucleus. After introduction of the vector into the developing new macronucleus during the sexual process of ciliates known as conjugation, the vector-borne micronuclear rDNA undergoes excision and is maintained as an rDNA minichromosome (Yao et al., Mol. Cell. Biol. 9:1092 (1989)).

Both somatic and processing rDNA vectors have been used to insert a heterologous nucleic acid into a 3' nontranscribed spacer region of rDNA. For example, M.-C. Yao et al. (Proc. Nat'l. Acad. Sci. USA 88:9493-9497 (1991)) expressed cycloheximide resistance in *Tetrahymena* using an rDNA vector having the rp129 cycloheximide resistant gene from *T. thermophila* inserted into the 3' nontranscribed spacer region (NTS) of the rDNA sequence. Similarly, P. Blomberg et al. expressed neomycin resistance in *T. thermophila* using an rDNA vector having the *neo* gene inserted into the 3' NTS, under control of rp129 flanking sequences (Mol. Cell. Biol., 17:7237-7247 (1997)).

Gaertig et al. described an rDNA-based shuttle vector, *E. coli* vector pH4T2, that contains two replication origin (ori) fragments, followed by a 300 base pair 5' untranslated region obtained from the *HHF1* gene of *Tetrahymena,* followed by the prokaryotic gene for neomycin resistance, *neo,* followed by a 3' untranslated region from *BTU2* from *Tetrahymena* (J. Gaertig et al., Nucleic Acids Res. 22:5391-5398 (1994)). Haddad et ale reported a small circular rDNA-based vector containing a repeat of the replication origin of rDNA (i.e., a 5' NTS), a *neo2* gene cassette (consisting of the *neo* gene under the control of histone *HHF1* promoter and the BTU2 transcription terminator) as a selectable marker, and a green fluorescent protein (GFP) cassette (also under control of *HHF1* promoter and BTU2 terminator) (A. Haddad et al., Proc. Nat'l. Acad. Sci. USA 94:10675-10680 (1997)). Rusconi et al. reported a circular vector containing the rDNA replication origin, *neo2* cassette, and a tRNA gene (Genes Dev. 10:2870-2880, 1996)).

A typical rDNA-based vector is a circular bacterial vector that contains a 5NTS comprising two or more of ori sequences from *Tetrahymena* rDNA, followed by a selectable cassette marker such as the *neo 2* cassette (Gaertig et al., Nucleic. Acids Res. 22:5391-5398 (1994). A nucleic acid fragment containing a heterologous coding region such as a transgene, flanked by a 5' untranslated region of a *Tetrahymena* gene (most often the ∼30 bp 5' untranslated region of the *HHF1* gene of *Tetrahymena*) and a 3' untranslated region of a Tetrahymena gene (most often ∼300 bp of the 3' untranslated region of the *Tetrahymena* gene *BTU2*)*,* is typically inserted downstream of the selectable marker.

An rDNA construct that contains relatively short 5' and 3' untranslated sequences from two different protein coding genes of *Tetrahymena,* such as *HHF1* and *BTU2,* is unlikely to integrate into the *Tetrahymena* genome via homologous recombination at the corresponding protein-coding loci. It is more likely to insert into *Tetrahymena* rDNA as a result of a single crossover event which involves the replication origin fragment. In addition, an rDNA-based vector can be maintained as an extrachromosomal element; the ori from *Tetrahymena* rDNA is known to support extrachromosomal replication. The marker gene (e.g., *neo*), and the transgene, if present, are therefore most likely expressed from the transforming rDNA-based plasmid and/or as a result of insertion into genomic rDNA, and not by recombination with endogenous genes other than rDNA.

Due to frequent and unpredictable integration of sequences from rDNA vector and rDNA-based vectors into the native rDNA, however, levels of expression of recombinant gene products are presumed to be highly variable. *See* J. Gaertig et al., Nucleic Acids Res. 22:5391-5398 (1994); R. W. Kahn et al., Proc. Natl. Acad. Sci. USA 90:9295-9299 (1993); W. J. Pan et al., Nucleic Acids Res. 23:1561-1569 (1995); and W. J. Pan et al., Mol. Cell Biol., 15:3372-3381 (1995). When relying on rDNA vectors for transformation, there is no way to control the level of integration into the host chromosome, hence no way to control copy number and, as a result, the expression level of a heterologous protein. *Tetrahymena* contain about 45 copies of each protein coding gene in the macronucleus, and each copy contains about 10,000 pallindromic copies per macronucleus. Thus, using either of these types of vectors, it is possible for a transgene to integrate at a similar copy number (10,000+). Overexpression of a transgene can be toxic to the protozoan host cell. Moreover, the loss of transgenes using these vectors cannot be prevented since this recombinant method generally lacks a reliable and sustainable means for selection. For example, a vector can contain both a transgene and a selectable marker, and both may initially integrate into the protozoan host genome. However, subsequent cross-over events can eliminate the transgene while leaving the marker gene in the host genome, resulting in selection of cells that do not necessarily contain the transgene.

A protein expression system that provides for the efficient expression and isolation of both prokaryotic and eukaryotic heterologous proteins in a nonpathogenic protozoan host is needed. In particular, a protein expression system that could provide surface expression of a heterologous prokaryotic or eukaryotic protein would constitute a much desired advance in the art.

### Summary of the Invention

The invention provides a protein expression system that utilizes a protozoan for the production of eukaryotic and prokaryotic polypeptides, including proteins. In one aspect, the recombinant protein expression system of the invention includes a transgenic protozoan host cell that is resistant to paclitaxel, wherein the host cell comprises a heterologous nucleic acid encoding a polypeptide. The recombinant protozoan host cell of this aspect of the invention is selectable by selection using paclitaxel. The protozoan host cell that is transformed with the heterologous nucleic acid is preferably a *Tetrahymena* host cell containing a *btu1-1K350M* β-tubulin allele. In another aspect, the recombinant protein expression system of the invention includes a transgenic ciliated protozoan host cell that contains a heterologous protein displayed on the plasma membrane surface of the host cell. Preferably, the surface-displayed heterologous protein is attached to the plasma membrane by a GPI anchor derived from an *Ichthyophthirius multifiliis* i-antigen.

Also provided by the invention is a novel protein expression vector. The vector contains a 5' flanking region followed by a heterologous nucleic acid encoding a polypeptide comprising at least one targeting amino acid sequence encoded by a portion of an i-antigen-encoding nucleotide sequence from *I. multifiliis,* followed by a 3' flanking region. At least a portion of each of the 5' flanking region and the 3' flanking region is complementary to an endogenous gene of an intended host, so as to allow for integration into endogenous gene by way of homologous recombination. In a preferred embodiment, the 5' flanking region and the 3' flanking region each contain a nucleic acid sequence selected from at least a portion of the *Tetrahymena* genes *HHF1, rpl29, BTU1, BTU2, SerH*3 and the gene encoding actin.

The invention further provides a transgenic *Tetrahymena* that contains at least a portion of an *I. multifiliis* i-antigen protein, preferably a portion of an *I. multifiliis* i-antigen protein that includes a targeting amino acid sequence.

Further, the invention provides a transgenic cell containing a heterologous protein that includes at least one targeting amino acid sequence encoded by an i-antigen-encoding nucleotide sequence from *1. multifiliis.* The transgenic cell is not limited and can be a bacterial cell, a fungus cell, a protozoan cell, or an animal cell, for example. The targeting amino acid sequence is an N-terminal targeting sequence, a GPI cleavage/attachment sequence, or both. Preferably, the the heterologous protein is displayed on the surface of the plasma membrane of the transgenic cell.

The invention also provides a method for making a polyclonal antibody. An antigenic polypeptide is expressed on the surface of the plasma membrane of a transgenic protozoan host cell, then the transgenic protozoan host cell is administered to an animal to generate an antibody response to the antigenic polypeptide. In another aspect of this embodiment of the invention, the antigenic polypeptide is cleaved from the surface of the host cell, isolated, then administered to an animal to generate an antibody response to the antigenic polypeptide. Optionally, the antibody is isolated from the animal.

The invention further provides a method for detecting antibodies to an antigenic polypeptide that involves expressing the antigenic polypeptide on the surface of a transgenic protozoan host cell; exposing the protozoan host cell to an antibody; and determining whether the protozoan host cell is immobilized. Immobilization of the protozoan host cell is indicative of the presence of antibodies to the antigenic polypeptide. This method can be used to detect antibodies to a pathogenic parasite in the bodily fluid of a patient suspected of being infected with the parasite.

Also provided by the invention is a method for screening drugs for the ability to bind a polypeptide. Preferably, the drug to be screened is one that has cross-linking capability, but the drug screening method of the invention is not limited to just those drugs. The polypeptide is expressed on the surface of a transgenic protozoan host cell and the host cell is exposed to the drug. Binding of the drug is evidenced by an observable change in the swimming pattern of the host cell which may, but need not, included complete immobilization of the host cell. In the case of a drug having cross-linking capability, the drug is caused to cross-link. Cross-linking of the drug will generally immobilize the host cell, indicating that the drug has bound to the polypeptide.

The invention further provides a vaccine containing a transgenic nonpathogenic immunogenic protozoan which exhibits a surface-displayed antigenic polypeptide. The vaccine can be a live vaccine or a killed vaccine. In a preferred embodiment, the nonpathogenic immunogenic protozoan is *Tetrahymena* and the antigenic polypeptide includes at least an antigenic portion of an *I. multifiliis* i-antigen protein.

The invention further includes a novel recombinant method for producing a polypeptide. A heterologous nucleic acid encoding the polypeptide is introduced into a protozoan host cell, preferably a protozoan host cell that is sensitive to paclitaxel, to yield a transgenic protozoan host cell selectable by negative selection using paclitaxel. The polypeptide is then expressed in the transgenic protozoan host cell. Optionally, the polypeptide is displayed on the plasma membrane of the transgenic protozoan host cell and cleaved to release the polypeptide. Also optionally, the polypeptide is isolated from the transgenic protozoan host cell. In another aspect, the recombinant method for producing a polypeptide includes introducing a heterologous nucleic acid encoding the polypeptide into a host cell to yield a transgenic host cell, wherein the polypeptide comprises at least one targeting amino acid sequence encoded by an i-antigen-encoding nucleotide sequence from *I. multifiliis,* then expressing the polypeptide in the transgenic host cell.

### Definitions

An "expression vector" is a nucleic acid molecule containing a nucleotide sequence encoding a polypeptide that is capable of being expressed in a host cell. Generally, when the term "vector" or "vector construct" is used herein, an expression vector is intended. Typically, an expression vector is a DNA molecule that contains a gene, and expression of the gene is under the control of regulatory elements that can, but need not, include one or more constitutive or inducible promoters, tissue-specific regulatory elements, and enhancers. Such a gene or other nucleic acid fragment is said to be "operably linked" to the regulatory elements.

A "cloning vector" is a nucleic acid molecule, typically a DNA molecule, that contains one or more restriction endonuclease recognition sites at which foreign nucleic acid fragments can be inserted in a determinable fashion without loss of an essential biological function of the vector, and often contain a nucleotide sequence encoding a detectable and/or a selectable marker (i.e., "marker genes") that can be used to identify and/or select cells transformed with the cloning vector. Marker genes typically include nucleic acid fragments that encode polypeptides which can confer a phenotypic characteristic to the transformed cell, such as antibiotic resistance, test compound metabolism, and the like.

Cloning and expression vectors can be include naturally occurring or modified DNA or RNA, and can take the form of a plasmid, cosmid, or bacteriophage. Vectors can be linear or circular.

The terms "exogenous" or "heterologous," which are used interchangeably herein, denote some item, typically a nucleic acid fragment or a protein, that is foreign to its surroundings. In particular, the terms apply to nucleic acid fragments that have been inserted into a host organism, but are not found in the normal genetic complement (i.e., genome) of the host organism. A nucleic acid fragment that is heterologous with respect to an organism into which it has been inserted or transferred is sometimes referred to herein as a "transgene." A "transgenic" organism (whether microorganism or an animal) is a host organism that has been genetically engineered to contain exogenous (heterologous) nucleic acid fragments, including vectors. Introduction of the heterologous nucleic acids into a host cell to create a transgenic cell is not limited to any particular mode of delivery, but includes, for example, microinjection, adsorption, electroporation, particle gun bombardment, liposome-mediated delivery and the use of viral and retroviral vectors.

Preferably, the heterologous nucleic acid fragments are stably integrated into the host genome, but they may, alternatively, be maintained extrachromosomally. The heterologous nucleic acid fragments may but need not be inheritable.

A nucleic acid fragment is "excised" from genomic DNA by isolating genomic DNA from the host, as by cutting the nucleic acid fragment at one or more predetermined sites, for example at a restriction enzyme recognition site.

An excised nucleic acid fragment can be ligated into a vector and assayed for recombination. Successful recombination can be detected, either directly or indirectly, as by using a laboratory assay or other detection procedure. Preferably, the presence or absence of a recombinant nucleic acid fragment is detectable by way of a chemical or biological assay. Detection can be mediated through the use of "reporter" nucleic acid fragments typically contained in the vector, such as a particular nucleic acid fragment that can confer metabolic pathways for particular nutrient utilization or antibiotic resistance. For example, in a *lac* operon-based mutation detection system commonly used in *E. coli, a* mutation in a *lac*I mutation target gene affects the expression of the *lac*Z reporter gene, and expression of the reporter gene is detectable in an *E. coli* host by assaying the ability of the host to produce the *lac*Z gene product (β-galactosidase) and thus metabolize a chromogenic substrate.

An "immunogenic" or "immune system stimulating" polypeptide, also referred to as a polypeptide antigen or an antigenic polypeptide, is one that activates at least one cell type of the immune system of an animal, including phagocytic cells such as macrophages, as well as T cells and B cells. An example of an immune-stimulating ciliated protozoan is one that displays a polypeptide antigen on its cell surface such that an immune response to the antigen is elicited in an animal exposed to the immune-stimulating protozoan. Likewise, an immunogenic composition comprises an immunogenic polypeptide and optionally contains a pharmaceutically acceptable buffer, such as phosphate buffered saline (PBS) or another buffer, recognized in the art as suitable and safe for introduction of proteins into a host to stimulate the immune system.

As used herein, the term "effective amount" refers to an amount of a substance that is effective to produce a desired result. For instance, an effective amount of an immune-stimulating composition is one that is effective to activate cells of the immune system, including phagocytic cells such as macrophages, as well as T cells and B cells. The actual amount varies depending upon the health and physical condition of a subject's immune system, i.e., to synthesize antibodies, the degree of protection desired, the formulation prepared and other relevant factors. It is expected that the amount will fall in a relatively broad range that can be determined through routine trials.

As used herein, the term "polypeptide" refers to a chain of amino acids linked through peptide bonds between an α-carboxyl carbon of one amino acid residue and the α-nitrogen of the next amino acid. The term polypeptide is used herein as a general term that includes polypeptides of any length, including what are commonly referred to in the art as peptides, oligopeptides, and proteins. A "protein" contains one or more polypeptide chains that fold to adopt a particular conformation that has some type of biological activity or function. The properties and function of any particular protein are generally determined by the physical and chemical properties of the molecule. The order of the nucleotides in a nucleic acid fragment determines the order of amino acid residues in a polypeptide and protein, i.e., the nucleic acid fragment "encodes" the polypeptide (protein).

### Brief Description of the Drawings

Figure 1 is a schematic of the vector pBICH3.
Figure 2 shows full restriction maps of (a) *BTU1::neol* construct (SEQ ID NO:1 and its complement, SEQ ID NO:2) and (b) pBICH3 vector construct (SEQ ID NO:3), as shown in as shown in Figure 1 (and its complement, SEQ ID NO:4); positions of restriction endonucleases sites are shown, with unique sites underlined; translation start and stop sites are boxed.

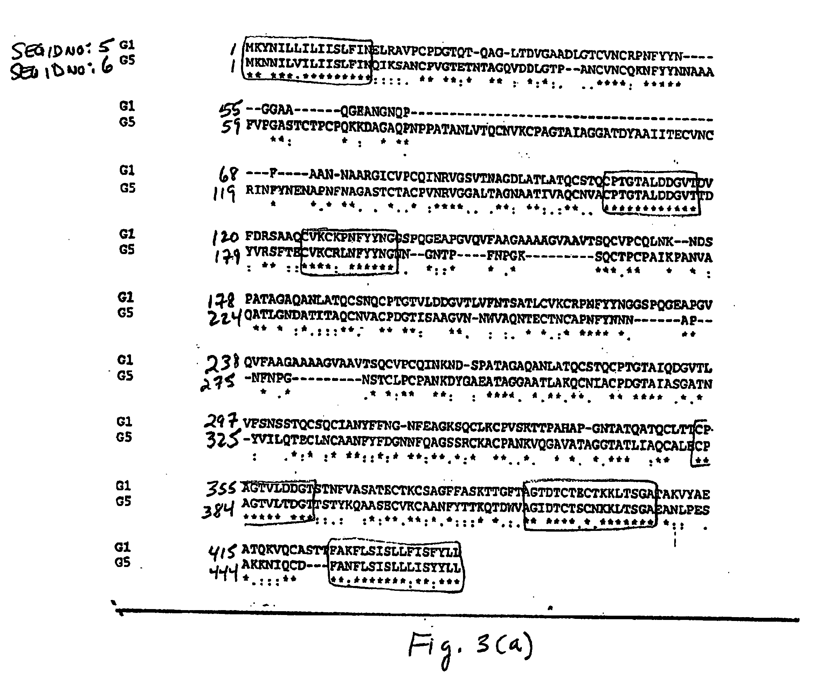

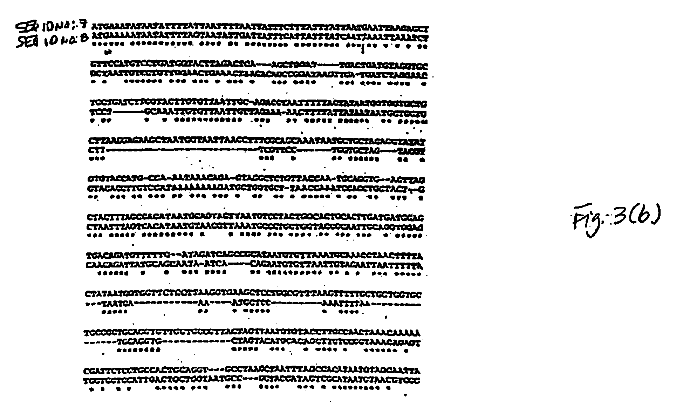

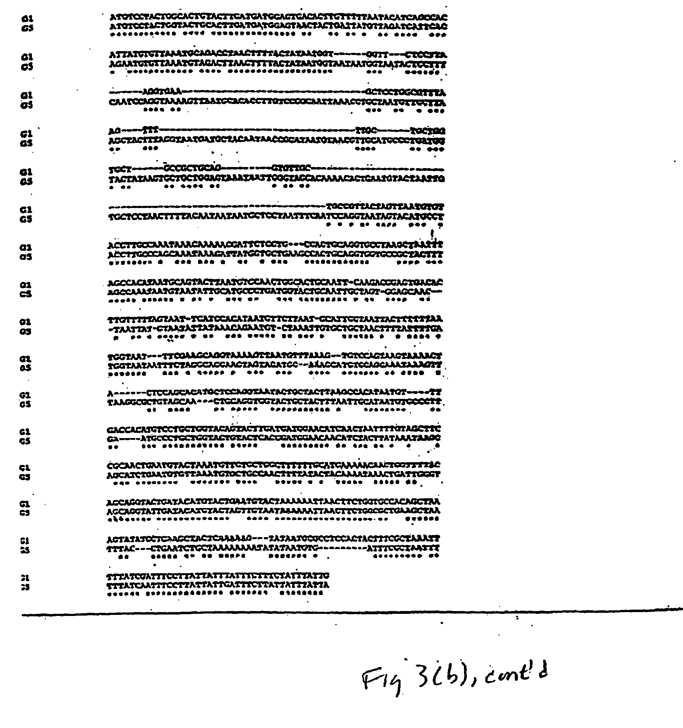

Figure 3 shows (a) an alignment of the deduced amino acid sequences of 48 kD (SEQ ID NO:5, upper line) and 55 kD (SEQ ID NO:6, lower line) i-antigens of *I. multifiliis,* where asterisks indicate identities between the two deduced protein sequences, double dots indicate highly homologous amino acids, and single dots indicate moderately homologous amino acids; boxes indicate conserved regions; (b) an alignment of the nucleotide sequences of the coding regions of the *IAG48 [G1]* gene (SEQ ID NO:7, upper line) and the *IAG55 [G5]* gene (SEQ ID NO: 8, lower line) of *I. multifiliis,* where asterisks indicate identities between the two nucleotide sequences.

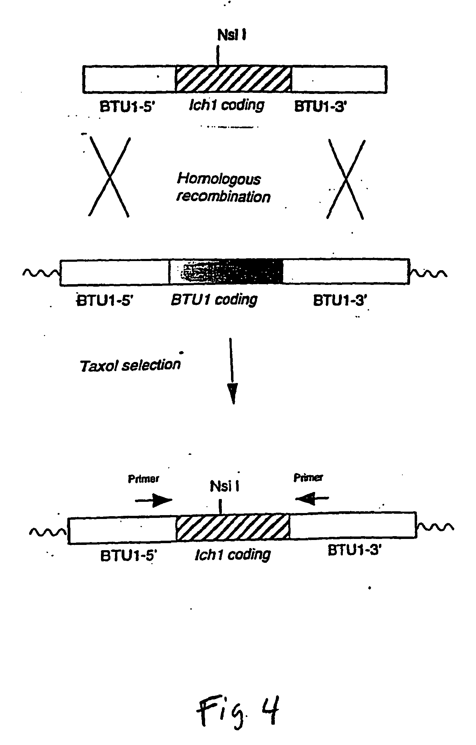

Figure 4 is a schematic showing the experimental design used to introduce the *Ichthyophthirius* i-antigen into the *BTU1* locus of *T. thermophila.* A hybrid gene composed of the *I. multifiliis* i-antigen coding sequence inserted between regulatory sequences of *BTU1* is integrated into the endogenous paclitaxel-sensitive *BTU1* locus by homologous recombination.
Figure 5 is a Western blot analysis identifying a putative 48 kD *I. multifiliis* i-antigen expressed by transgenic *T. thermophila.*
Figure 6A-6D are photomicrographs using immunofluorescence for detection of surface expression of a putative 48 kD *I. multifiliis* i-antigen in transgenic *T. thermophila.*
Figure 7 shows Western blots of (a) total protein extracts of transformant cells; and (b) total culture medium from which transformed cells were removed, probed using anti- *I. multifiliis* i-antigen antibodies; the load of the medium shown in (b) is about 1/30th of the load of the cells shown in (a).
Figure 8 shows a Western blot of a total protein extract from *Tetrahymena* transformed with chicken ovalbumin, probed with anti-chicken ovalbumin antibodies.
Figure 9 is Western blot showing expression and secretion from a transformed *Tetrahymena* of a G 1 i-antigen with C-terminal truncation.
Figure 10 shows ELISA results for fish vaccinated with *Tetrahymena* expressing (a) membrane associated G1 i-antigen and (b) secretary form of G1 i-antigen.
Figure 11 is a 10X magnification of serotype A *I. multifiliis* an immobilization test conducted using sera (1:20 dilution) from (a) fish vaccinated with live *Tetrahymena* expressing *neo* ("anti-live Tneo," the negative control) and (b) fish vaccinated with live *Tetrahymena* expressing the full-length 48 kD i-antigen protein from a G1 *I. multifiliis* isolate ("anti-live TG1").

### Detailed Description

The invention provides a protein expression system that utilizes a protozoan, preferably a nonpathogenic ciliate, more preferably *Tetrahymena,* for the production of eukaryotic and prokaryotic polypeptides, including proteins. The protein expression system of the invention is useful to produce virtually any polypeptide for any purpose. It is suited to both large scale and analytical scale production of recombinant polypeptides, and is particularly useful for expression of polypeptides that are difficult to produce in conventional recombinant protein expression systems.

### Ciliated protozoa

Protozoa, particularly nonpathogenic protozoa, are well-suited for use as recombinant protein expression systems. Not only are many protozoa capable of secreting proteins, but they often contain an anchoring system (known as a GPI anchor) that allows surface expression, or "display," of various endogenous proteins without secretion. Some protozoa, like *Tetrahymena,* have major membrane proteins that are GPI-anchored; they in essence contain a GPI-anchored coat of surface-displayed proteins on the plasma membrane. These displayed proteins can often be cleaved from the cell surface of a protozoan by phospholipase C (typically isolated from *B. thuringiensis*). Many nonpathogenic protozoa are fast-growing and inexpensive to culture. Nonpathogenic ciliated protozoa can be environmentally friendly if the desired genetic modifications are performed within their somatic genomes localized to the macronucleous; heterologous nucleic acids that have integrated into the macronucleus, such as those that disrupt the *BTU1* gene in *Tetrahymena,* are lost when the protozoan undergoes the sexual process of conjugation. The old macronucleus disintegrates, and the new macronucleus is formed as a result of differentiation of the zygotic micronucleus. As a result, a nucleic acid that had integrated into the macronucleus is not inherited or otherwise perpetuated, a feature which enhances environmental safety by preventing heterologous nucleic acids from entering the gene pool.

Protozoa are also more effective than yeast in providing post-translational modifications to certain eukaryotic proteins. For example, yeast apparently lacks a number of post-translational modifications typically found on tubulins, such as acetylation of Lys40 in α-tubulin, polyglutamylation and polyglycylation of α-and β-tubulin (T. MacRae et al., Eur. J. Biochem. 244:265-278 (1997)). On the other hand, these tubulin modifications were found in *Tetrahymena* and other ciliates like *Paramecium* (J. Gaertig et al., J. Cell Biol. 129:1301-1310 (1995)); V. Redeker et al., Science 266:1688-1691 (1994)). Ciliates, in particular, are excellent candidates for heterologous polypeptide expression; the presence of cilia vastly increases cell surface area, providing more opportunity for expression of GPI-anchored polypeptides.

A transgenic nonpathogenic ciliated protozoan of the invention is preferably a free-living ciliate; that is, it can propagate without a host. Examples of nonpathogenic free-living ciliates include *Tetrahymena, Paramecium, Blepharisma, Colpidium, Euplotes, Stylonichia* and *Oxytricha.* More preferably, the free-living transgenic nonpathogenic ciliate is *Tetrahymena. Tetrahymena* can be grown in large volume cultures using a variety of inexpensive media including skimmed milk powder. Generation time is short (1.5-3 hr) and cells attain remarkably high density given their size (∼50 µm in length). Indeed, it has been reported that fermentation in perfused bioreactors has permitted growth to concentrations as high as 2.2 X 10⁷ cells/ml, equivalent to a dry weight of 48 g/l. See, e.g., T. Kiy et al., Appl. Microbiol. & Biotech., 38:141-146 (1992). Further, *Tetrahymena* has the ability to secrete proteins into the growth medium, and mutant strains defective in the release of hydrolytic enzymes have been isolated (P. Hunseler et al., Dev. Genet.. 13:167-173 (1992). Mutant strains lacking or exhibiting reduced levels of secreted hydrolytic proteases are preferred for surface expression or secretion of a heterologous polypeptide that is sensitive to proteolysis by hydrolytic enzymes. Finally, a large part of the cell metabolism is devoted to the production of abundant surface membrane proteins known as immobilization antigens (i-antigens), whose expression is tightly regulated by environmental conditions. This feature makes *Tetrahymena* a preferred host because expression of a heterologous protein containing an endogenous GPI anchor can potentially be controlled by changes in temperature. J.R. Preer, The Molecular Biology of Ciliated Protozoa, pp. 301-339 (ed. J.G. Gall), Academic Press, New York (1986).

*Tetrahymena,* which has genomic DNA generally on the order of 75% AT, offers additional advantages as a host system for the cloning and expression of genes from other organisms with AT-rich genomes. For example, a number of human pathogens (for example, malarial plasmodia, mycoplasmas, etc.) have extremely AT-rich genomes. Genomic DNA contains four bases (A, T, C, G), and DNA duplexes typically hybridize by means of A-T and G-C base pairing. In the genomes of many organisms, the amounts of A, T, C, and G are relatively equal, thus their genomic DNA contains roughly the same number of A-T and G-C base pairs. "AT-rich" genomes or nucleic acids are genomes or nucleic acids that have an AT content of more than about 50% of the total bases in the fragment, preferably about 65% or more, and more preferably about 70% or more. Notably, AT-rich fragments are inherently unstable in conventional systems such as *E. coli,* making cloning genes from AT-rich organisms into conventional systems difficult. As an AT-rich host, however, *T. thermophila* is expected to stably maintain heterologous nucleic acid fragments having "AT rich" sequences.

*Tetrahymena,* like other ciliates, utilizes UAA and UAG as codons for the amino acid glutamine, while most other organisms recognize those as termination codons. Thus, *Tetrahymena* is particularly useful to express heterologous genes that are derived from other ciliates. Expression of ciliate genes in conventional (eukaryotic and prokaryotic) protein expression systems often requires that the ciliate gene be mutated so as to utilize the conventional nucleic acid code in order to produce a full-length protein.

Furthermore, immobilization of *Tetrahymena* in culture with specific antibodies offers a potential bioassay for detection of specific antibodies in biological samples that would shorten the time and lower the cost for screening, and would obviate the need for chemical detection substrates used in more standard diagnostic tests.

### Transformation of the protozoan host with a heterologous nucleic acid

The protozoan host is transformed with a heterologous nucleic acid, which can be either integrated into the host's genome or maintained extrachromosomally on an autonomous plasmid or other construct. The heterologous nucleic acid encodes a polypeptide that is expressed by the resulting transgenic host. There is no limitation on the type of heterologous polypeptide that can be expressed in the protozoan host. The polypeptide coding region of the vector construct can, for example, be a coding region derived from a pathogenic protozoan, or from another eukaryotic or prokaryotic organism. As already noted, a protozoan host such as *Tetrahymena* is especially useful as a vehicle for creating an expression library for organisms with AT-rich genomes, particularly pathogenic organisms, such as *Plasmodium* (the protozoan agent of malaria). Genomic fragments from these organisms are difficult to clone and stably maintain in *E. coli.* Another example of a preferred heterologous nucleic acid sequence is one that encodes at least a portion of an antigenic polypeptide in that is capable of stimulating an immune response in an animal upon exposure to the polypeptide. In a particularly preferred embodiment of the invention, a transgenic ciliated protozoan displays an antigenic polypeptide on its cell surface.

The location of the expressed polypeptide is determined by the protein targeting sequences, if any, encoded by the heterologous nucleic acid and thereby incorporated into the resulting polypeptide. The heterologous polypeptide can accumulate in the cytosol, can be secreted from the host cell, or can be embedded in or anchored to the host cell plasma membrane, as is further described below.

Preferably, the heterologous nucleic acid is introduced into the ciliate host on an expression vector that is capable of integrating into the host's genome. In a particularly preferred embodiment of the invention, expression vectors operate by way of homologous recombination with a highly expressed gene that is endogenous to the protozoan host, such as a β-tubulin gene as described below. On the other hand, an expression vector that does not rely on the regulatory sequences of an endogenous gene for expression can, optionally, be maintained extrachromosomally in the ciliated protozoan host cell. An expression vector maintained as an extrachromosomal element is preferably an rDNA-based vector containing ori from *Tetrahymena* rDNA, which is known to support extrachromosomal replication. Such a vector further includes a 5' regulatory region from an endogenous *Tetrahymena* gene containing a promoter region operably linked to the heterologous coding region and, optionally, a 3' regulatory region from the same or, preferably, a different *Tetrahymena* gene. For example, expression vectors can contain regulatory regions from ciliate genes such as *HHF1, rpl29, BTU1, BTU2, SerH3,* and those encoding actin.

### Expression vectors

Expression vectors useful for transforming protozoa in accordance with the invention can be generally classified into three types: replacement vectors, rDNA vectors, and rDNA-based vectors. Replacement vectors accomplish DNA-mediated transformation by replacing or altering endogenous genes using homologous recombination. Integration of the heterologous nucleic acid into the host's genome at the targeted site is accomplished via homologous recombination involving a double crossover event with the vector containing the heterologous nucleic acid. An example of an expression vector useful for genomic incorporation of a heterologous nucleic acid by replacement is one that includes a heterologous coding sequence flanked by portions of the endogenous *BTU1* gene of *Tetrahymena.*

A replacement vector thus preferably includes a 5' region, followed by a heterologous coding region, followed by a 3' region, wherein at least a portion of each of the 5' and 3' regions is complementary to 5' and 3' regions on an endogenous gene of the host, to allow for genomic integration of the heterologous coding region via homologous recombination. The 5' and 3' regions of the vector optionally contain regulatory elements, such as a promoter and a terminator; alternatively, the necessary regulatory elements are supplied by the endogenous gene into which the heterologous coding region integrates. Preferably, the 5' and 3' regions of an expression vector useful in *Tetrahymena* include sequences complementary to the *BTU1, BTU2, SerH3* or *HHF1* genes of *Tetrahymena.* Optionally, a replacement vector further includes a nucleotide sequence encoding a selectable marker, such as *neo.* Preferably the marker sequence is under the control of its own promoter, thus another regulatory region is included 5' to the sequence encoding the selectable marker; and example is the *neo2* cassette.

β-Tubulin is a cytosolic protein in *Tetrahymena* that is a monomeric constituent of cytoskeletal elements known as microtubules. An α/β tubulin dimer is the building block of microtubules, and both α-tubulin and β tubulin are essential proteins. *T. thermophila* expresses two major β-tubulin genes, *BTU1* and *BTU2,* which encode identical β-tubulin proteins (J. Gaertig et al., Cell Mot. Cytoskel., 25:245-253 (1993)). It was determined that either of these two genes (but not both at once) can be disrupted without a detectable change in the cell phenotype.

Substitution of lysine (K) 350 by methionine (M) in the *BTU1* gene to yield the *Btul-1K350M* allele confers increased resistance to several microtubule-depolymerizing drugs (oryzalin, colchicine, vinblastin), and increased sensitivity to a microtubule-stabilizing agent, paclitaxel (J. Gaertig et al., Proc. Nat'1. Acad. Sci. USA 91:4549-4553 (1994)). The phenotype of the *Btul-1K350M* allele is expressed even in the presence of wild-type copies of the second β-tubulin gene, *BTU2.* Cells carrying the *btu1-1K350M* allele can be transformed to paclitaxel resistance by gene replacement of *btu1-1K350M* with a wild-type *BTU1* gene fragment, thus eliminating the need to incorporate a means for positive selection. Because the *BTU1* gene is not essential for survival, any loss-of function mutation of *btu1-1K350M* in the presence of wild-type *BTU2* gene confers paclitaxel resistance.

It has been discovered as part of this invention that heterologous nucleic acids can be inserted into a β-tubulin gene of *T. thermophila* for successful cell-surface expression that is advantageously maintained by way of selection. Expression of a heterologous protein in *T. thermophila* is preferably accomplished using a transgenic *T. thermophila* that contains the negatively selectable *Btul-1K350M* allele of the β-tubulin gene (*BTU1*) to direct heterologous nucleic acid fragments to this highly expressed locus by homologous recombination. A transformed cell line can thus be readily identified by paclitaxel selection, in that successful transformation restores paclitaxel resistence.

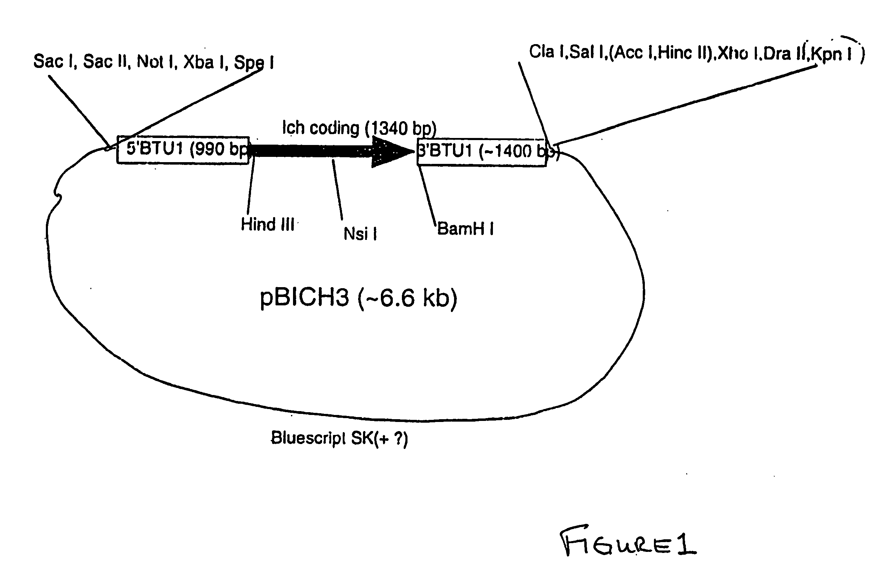

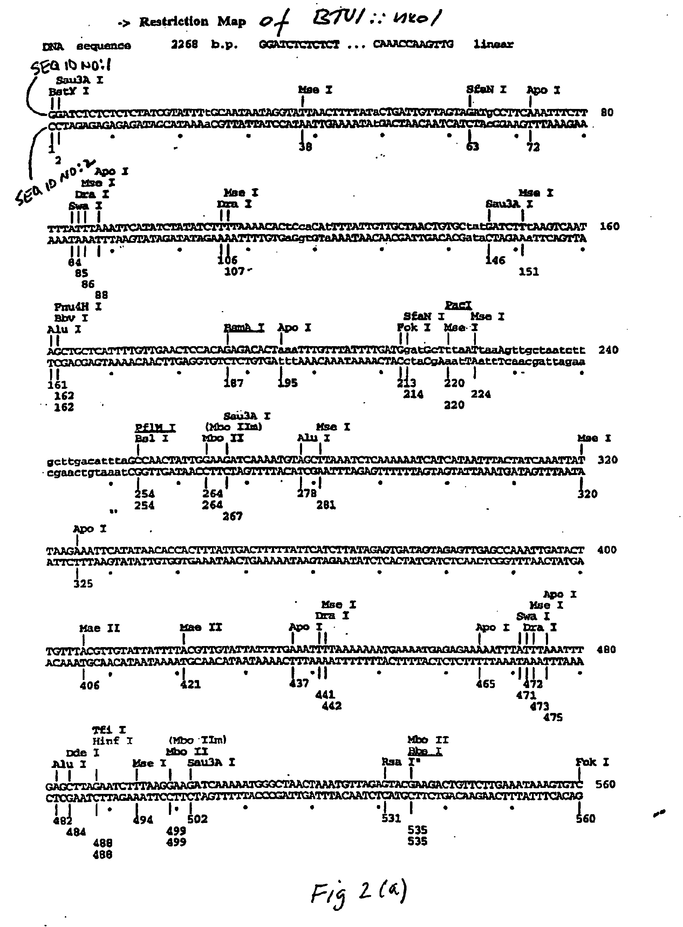

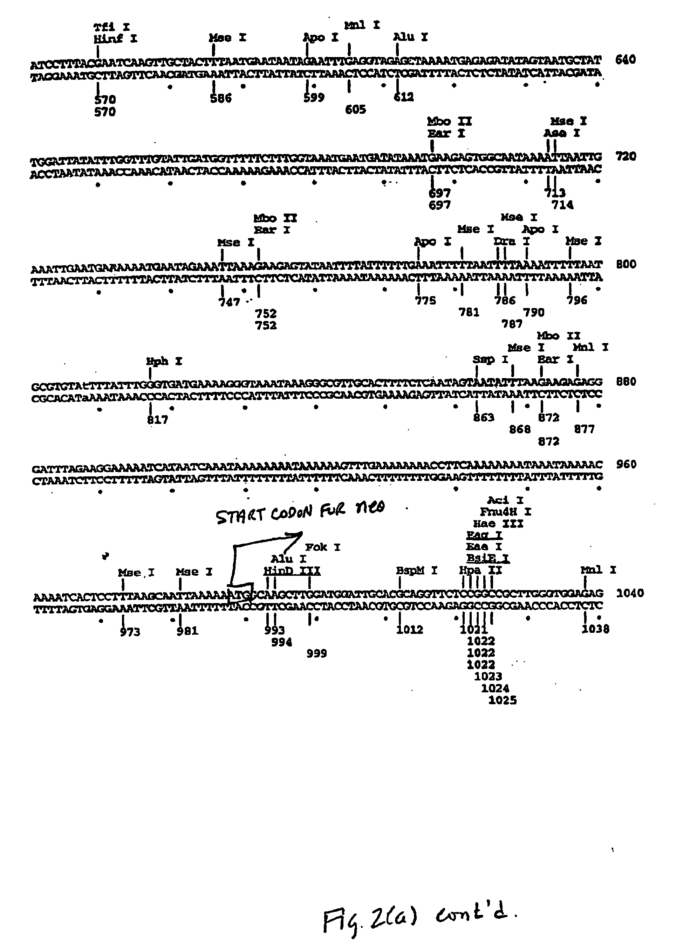

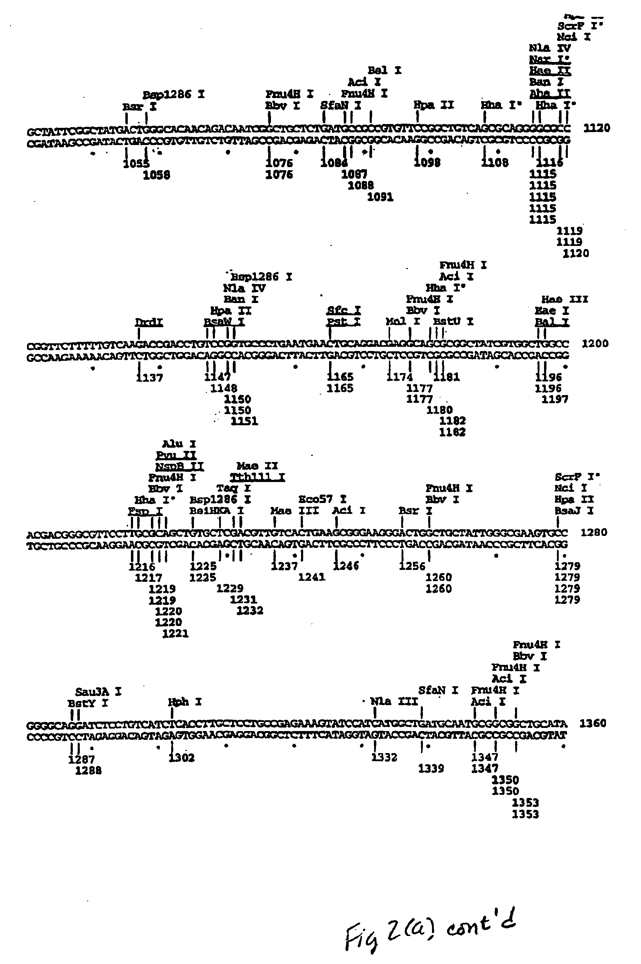

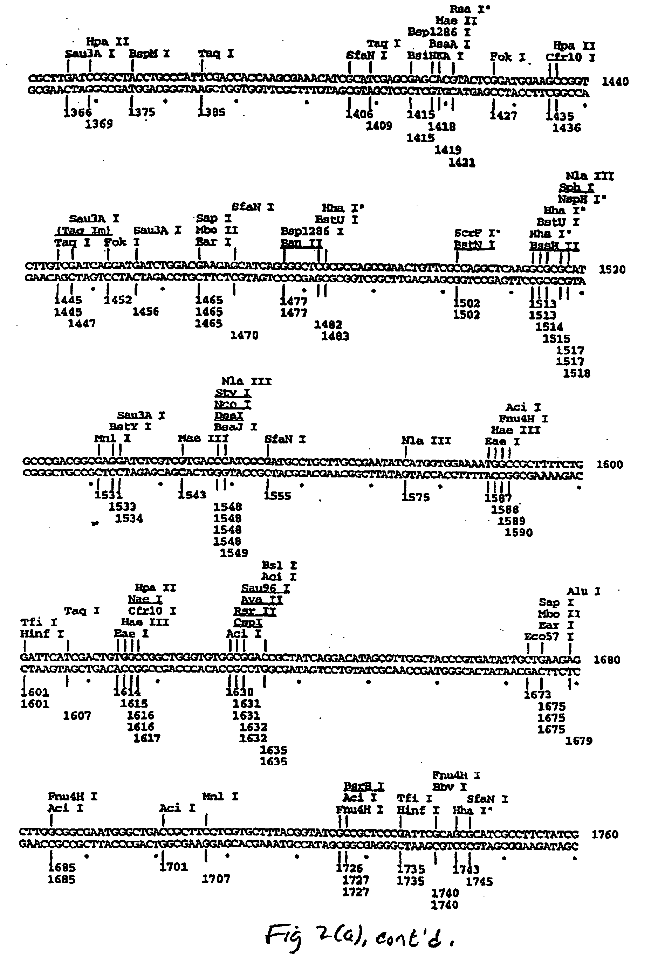

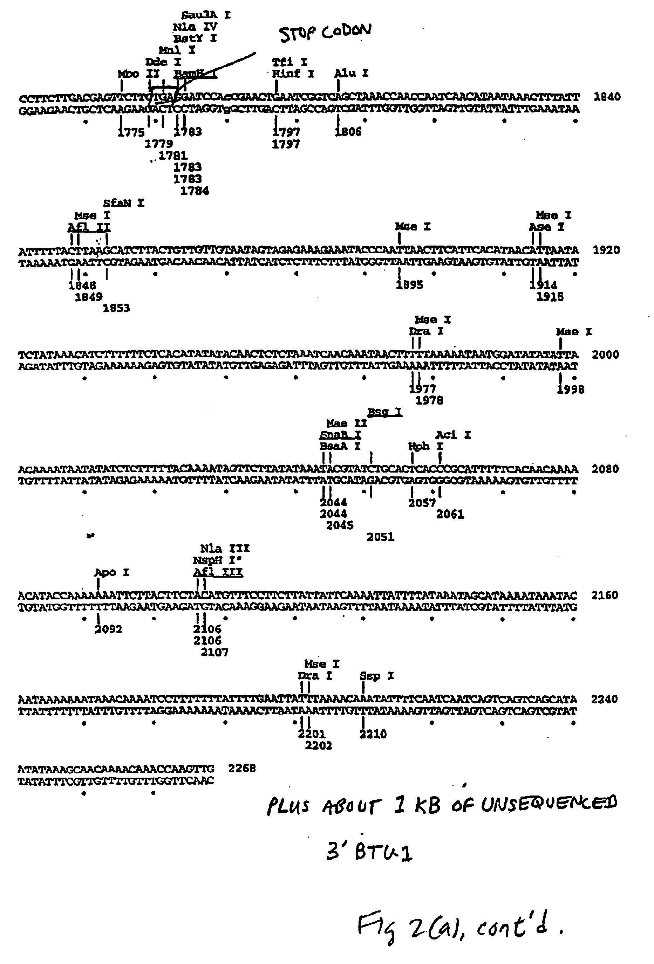

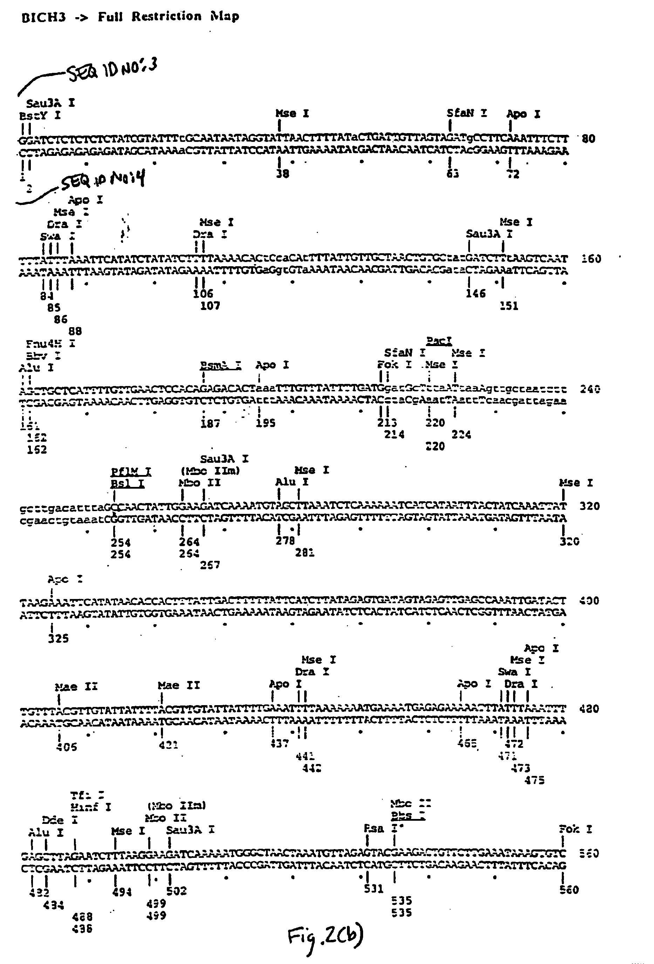

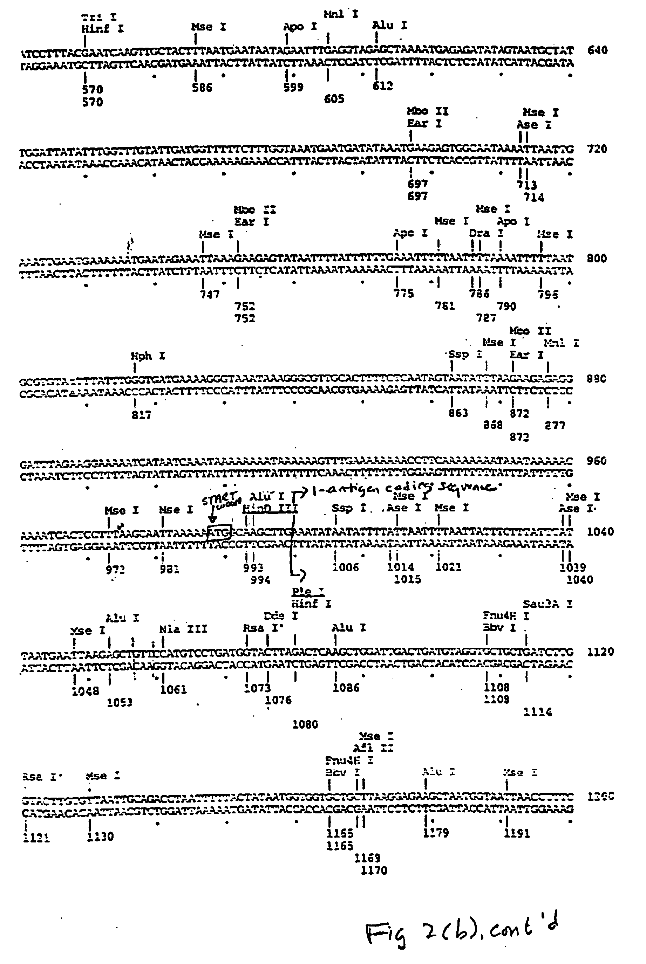

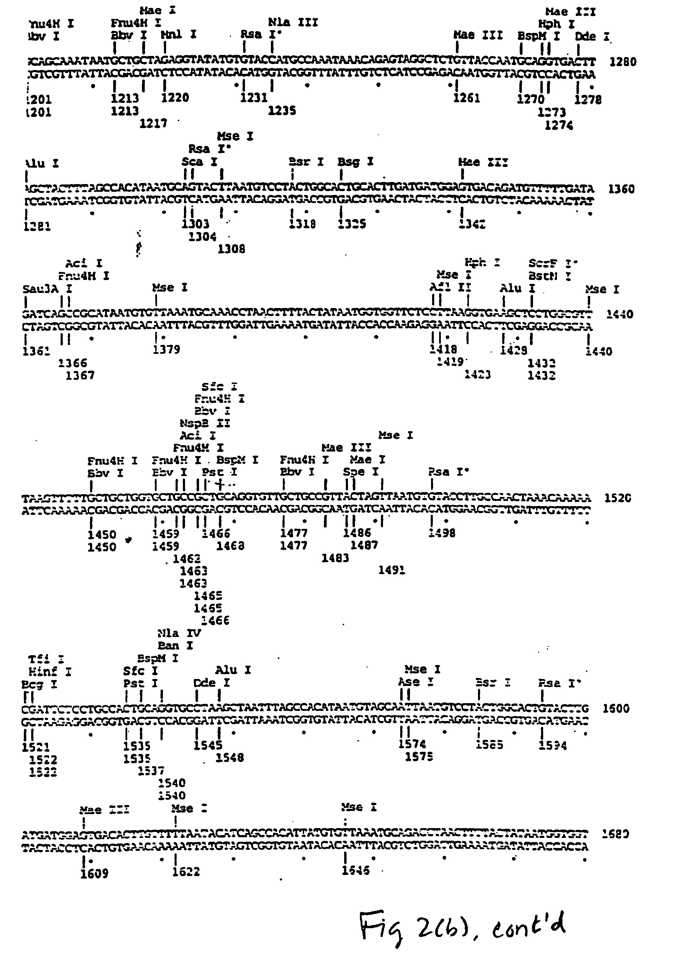

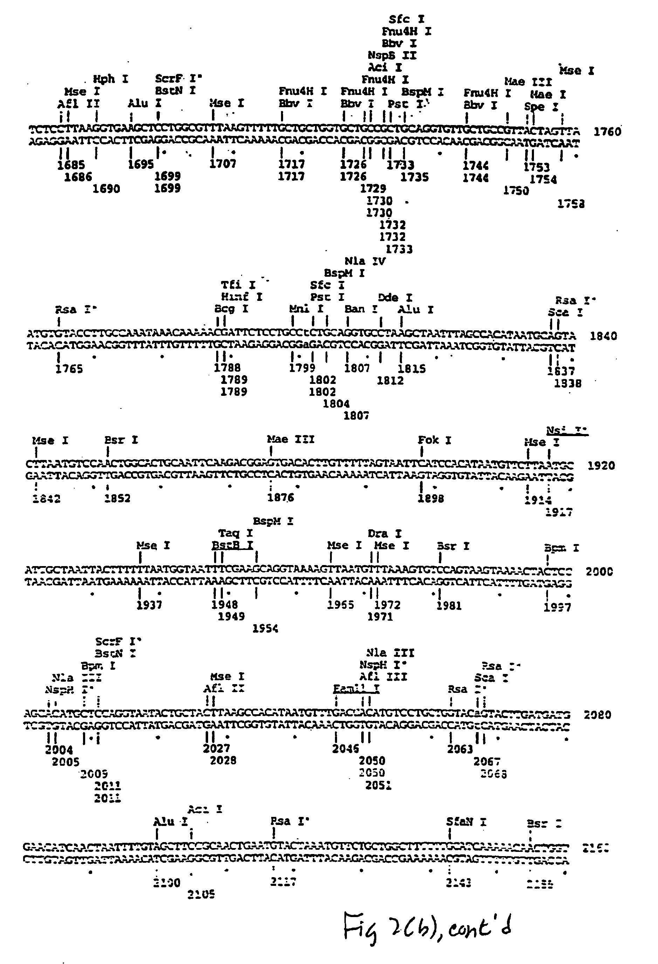

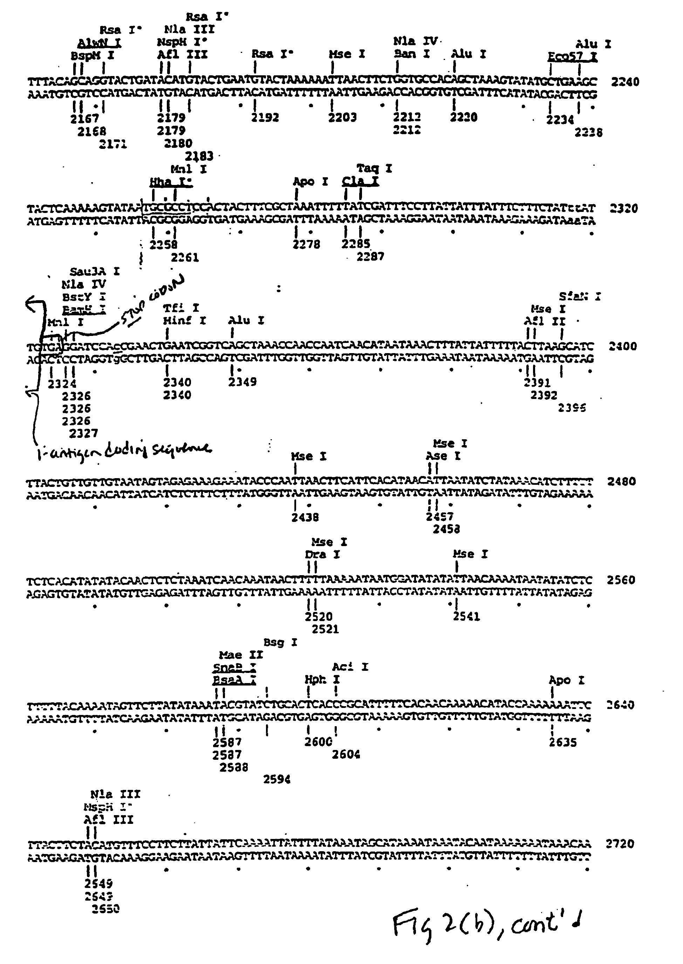

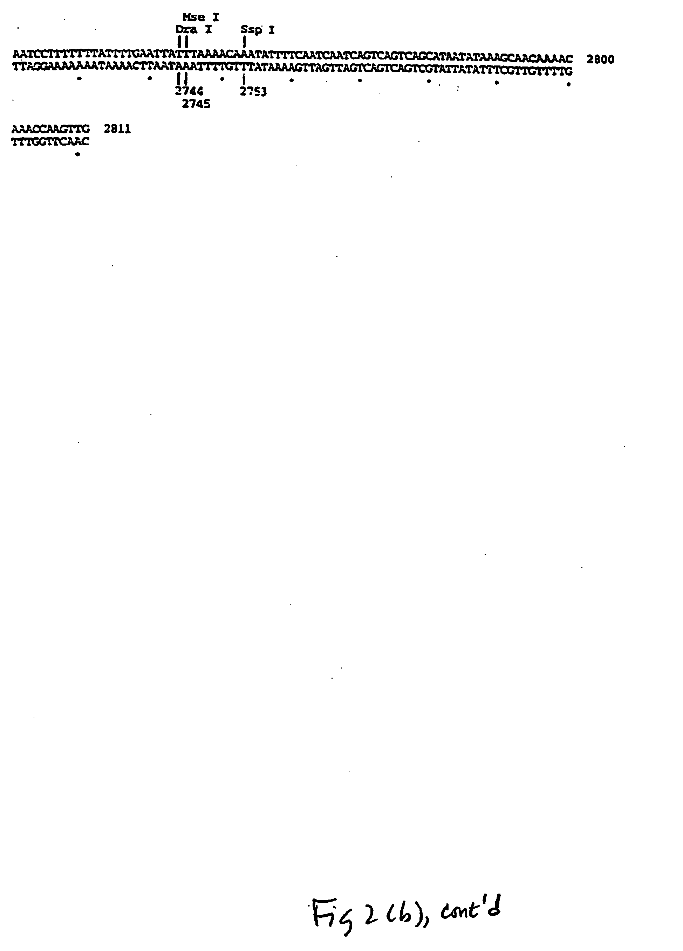

Accordingly, a preferred expression vector in accordance with the present invention preferably includes a nucleic acid fragment capable of integration into either the *BTU1* or *BTU2* gene of *T. thermophila,* preferably the *BTU1* gene, more preferably the *Btu1-1K350M* allele of the *BTU1* gene. The *BTU1* gene contains 5' and 3' regulatory regions that flank the coding region; hence the preferred expression vector contains at least a portion of each of these regulatory regions, which portions are sufficient to allow for homologous recombination with the endogenous gene. The flanking sequences of *BTU1* that are included in the vector (e.g., Figs. 1 and 2) allow for targeting of the heterologous coding region into the endogenous *BTU1* gene via homologous recombination with the endogenous gene.

The protozoan host to be transformed with the vector is preferably *T. thermophila* which contains a β-tubulin gene (*BTU1*) wherein lysine 350 is substituted by methionine (*btu1-1K350M*)*.* The expression vector disrupts the *btul-1K350M* gene by homologous recombination-mediated insertion of a heterologous nucleic acid, thereby restoring resistence to paclitaxel in the resulting transgenic host. Homologous recombination can take place at sites within the coding region of the highly expressed target gene or at sites that flank the gene target, which include but are not limited to flanking regulatory regions; all that is required is that the expression of the target gene be disrupted. When *BTU1* is the target gene, a second β-tubulin gene (*BTU2*) remains available to provide the essential function of β-tubulin *in vivo,* such that *BTU1* can be fully replaced with *btu1-1K350M* (or heterologous nucleic acid sequence) without adverse effects to the organism. When *T. thermophila* having the *btul-1K350M* allele of the *BTU1* gene is used as the host, transformants can be maintained by selection, since transformed mutants are not sensitive to paclitaxel while nontransformed mutants are. Transformants are this readily identified by selection by growth on in media containing paclitaxel; there is no need to engineer drug-resistance or other means of selection into the genome of the transgenic host. Additionally, there is no limitation on the nature of the heterologous nucleic acid sequence that can be targeted to the *BTU1* locus of *T. thermophila.*

The heterologous nucleic acid sequence that is incorporated into the host genome in accordance with the present invention optionally encodes a selectable marker. For example, an expression vector can include a *BTU1* derivative, *BTU1-2::neol* (see Fig. 2) which substitutes the coding region of a prokaryotic gene, *neo1,* for that of *BTU1,* to provide a means for positive selection of transformants. The *neol* gene confers resistance to paromomycin in *T. thermophila,* and a *BTU1-2::neol* fragment can then be used to generate *BTU1* gene knockouts by homologous recombination and positive selection with paromomycin. Of course, when the host organism is a *T. thermophila* mutant containing the *btu1-1K350M* allele of *BTU1,* transformants are identified with positive selection.

Optionally, a replacement vector for use in *Tetrahymena* includes a temperature-regulatable promoter region to facilitate controlled expression of the heterologous coding region. The temperature-regulatable promoter can be supplied instead of or in addition to another promoter, such as the promoter region of the endogenous *BTU1* gene. The temperature-regulatable promoter is preferably positioned within the 5' untranslated region of the vector, more preferably between a 5' BTU region and the heterologous coding region. Because *Tetrahymena SerH3* encodes an i-antigen whose expression is temperature dependent, the 5' regulatory region of the *SerH3* gene of *Tetrahymena* may include a temperature-regulatable promoter that is useful in the invention.

Also optionally, the replacement vector contains a second promoter region, in addition to the promoter region supplied by the endogenous target gene, for enhanced expression of the heterologous protein.

In a representative example of the invention, the expression vector (pBICH3, Fig. 1) includes the entire coding sequence of the i-antigen gene inserted in a correct translational frame between the regulatory sequences of the *BTU1* gene. This vector contains a complete plasmid sequence commercially available under the trade designation pBLUESCRIPT SK (+), from Stratagene, La Jolla, CA, and is generally useful for integration of any heterologous coding region into the β-tubulin gene of *T. thermophilis.* One skilled in the art will recognize that other plasmids may be equally suitable, such as those commercially available under the trade designation pCRScript, from Stratagene, La Jolla, CA; pGEM from Promega, Madison, WI; and pCRII, from Invitrogen, Carlsbad, CA.

Figure 2(b) shows the full restriction map of pBICH3, represented by SEQ ID NOs:3 and 4. The complete nucleic acid sequence (SEQ ID NO:7) and deduced amino acid sequence (SEQ ID NO:5) of the DNA encoding the 48 kD *Ich* i-antigen is presented in Figure 3. Nucleic acid sequence SEQ ID NO:7 of Figure 3(b) is included in pBICH3 at base pair 1000 to base pair 2325 as shown in Figure 2(b). The fragment GCAAGCTTG at base pair 991 to 999, which follows the start codon ATG and immediately precedes the i-antigen coding sequence, is a cloning residual from the parent construct, HHF1::neo (R. Kahn et al., Proc. Nat'1. Acad. Sci. USA 90:9295-9299 (1993)). The resulting vector construct can be propagated in *E. coli* as a shuttle vector as well.

Gene regulatory elements useful in such transformation systems include both upstream (5') and downstream (3') regulatory elements. For example, in prokaryotic and eukaryotic genes, the upstream region contain promoter elements that specify correct initiation of mRNA synthesis. Although classical promoters have not yet been identified in ciliates, examination of the upstream regions of various ciliate genes shows the presence of sequence elements that are highly conserved, and which are known to be required for gene expression in a wide variety of other eucaryotic systems. For example, TATA sequences that play a critical role in transcription initiation in most eukaryotes, are present in the 5' flanking regions of a number of ciliate genes (for example, Cupples et al. (1986) Proc. Natl. Acad. Sci. USA 83:5160; Brunk et al. (1990) Nucl. Acids Res. 18:323; Tondravi et al. (1990) Mol. Cell. Biol. 10:6091; Prat et al. (1986) J. Mol. Biol. 189:47). Less common eucaryotic promoter elements (consisting of CCAAT sequences) are also present. The upstream regions of ciliate genes contain information that is both necessary and sufficient for promoter function.

Because promoter elements are subject to differential regulation and have profound effects on RNA transcription levels *in vivo,* different promoters can be used for the expression of the heterologous nucleic acid. These include the promoters from the actin, histone H4 and *SerH3* i-antigen genes from *Tetrahymena thermophila* (Cupples et al. (1986) Proc. Natl. Acad. Sci. USA 83:5160; Brunk et al. (1990) Nucl. Acids Res. 18:323; and Tondravi et al. (1990) Mol. Cell. Biol. 10:6091). Expression from the actin promoter is typically constitutive, while expression from the histone and *SerH3* promoters are cell-cycle and temperature-dependent, respectively. Expression vectors capable of autonomous replication must include a promoter element; however, when the heterologous nucleic acid is genomically integrated, inclusion of a heterologous promoter element in the construct is optional, as the 5' regulatory region of the endogenous gene can be utilized instead.

The sequences required for correct 3' processing of mRNA transcripts in ciliates (that is, transcription termination and polyadenylation) lie in close proximity to (and approximately 100 bp on either side of) the 3' terminus of most ciliate RNA transcripts. For example, an additional region from the 3' end of the *SerH3* i-antigen gene from *T. thermophila* can be added to the heterologous nucleic acid prior to cloning and expression.

rDNA vectors and rDNA-based vectors, including the typical components of each, were discussed in detail in the background. Although not preferred embodiments of the invention, these vectors can nevertheless be effectively used to express heterologous proteins in *Tetrahymena.* Depending upon the vector design, rDNA vectors and rDNA-based vectors can integrate into the host genome, for example via homologous recombination or a single cross-over event, or they can be maintained extrachromosomally. Either type of vector can be constructed to include a heterologous protein containing an N-terminal targeting sequence, a C-terminal targeting sequence (i.e., a GPI anchor) or both, for targeted expression of a heterologous protein in Tetrahymena and other ciliates. Preferably, the N-terminal targeting sequence and/or the C-terminal targeting sequence is derived from an i-antigen of *I. multifiliis.*

### Targeted protein production

Advantageously, the invention allows the recombinant production of a heterologous polypeptide to be targeted within or outside of a cell. The targeting mechanism is not limited to protozoan hosts but can be used in any type of cell, for example a mammalian cell, a yeast cell, a protozoan cell, or a bacterial cell. Preferably, however, the cell is a cell of a ciliated protozoan. Optionally, therefore, a recombinant genetic construct of the invention (i.e., expression vector or transgenic organism) contains a heterologous nucleic acid that encodes a protein comprising an N-terminal protein targeting sequence or a C-terminal protein targeting sequence, or both. An N-terminal targeting sequence directs a protein to the endoplasmic recticulum (ER), and as a result can cause the protein to embed in the plasma membrane or to be secreted out of the cell. A C-terminal targeting sequence, specifically a GPI anchor, can cause the protein to be displayed on the membrane surface of the host cell. These targeting sequences can be either endogenous to the host cell, as where the heterologous nucleic acid is introduced into the host's genome by way of homologous recombination with a native coding sequence, or heterologous to the host cell.

Thus, to direct a heterologous polypeptide to the plasma membrane surface ("surface display"), the vector construct preferably encodes a heterologous polypeptide comprising (1) an amino terminal signal peptide and (2) a GPI cleavage/attachment sequence (i.e., a "GPI anchor"). The expression system of the invention is thus uniquely suited for expression of membrane proteins such as receptor proteins or proteins that utilize a GPI anchor. In other applications in which secretion of a heterologous polypeptide is intended, the vector construct preferably encodes a heterologous polypeptide comprising an amino terminal signal peptide but not a GPI cleavage/attachment sequence. Finally, for intracellular production of the heterologous polypeptide, the heterologous polypeptide encoded by the vector construct preferably includes neither an amino terminal signal peptide nor a GPI cleavage/attachment sequence.

Surprisingly, surface display of heterologous proteins in *Tetrahymena* is not limited to the use of a GPI anchor modification domain that is native to *Tetrahymena.* It has been discovered, for example, that proteins that contain a GPI anchor derived from an immobilization antigen ("i-antigen") of *Ichthyophthirius multifiliis* are displayed on the membrane surface of *Tetrahymena* (Example II). Likewise, it is not necessary to use N-terminal signal peptides derived from *Tetrahymena;* it is shown in Example VI that the N-terminal signal peptides of both I. *multifiliis* i-antigen and *T. cruzi* gp-72 protein work well in *Tetrahymena.*

Thus, protein targeting sequences can be derived, for example, from *I. multifiliis, T. cruzi, Paramecium, Dictyostelium, Leishmania, Toxoplasma, Crithidia, Plasmodium* and *Chlamydomonas.* An example of an N-terminal protein targeting sequence from *T. cruzi* is the N-tenninal signal peptide of the gp-72 surface protein. Heterologous protein targeting sequence obtained from *I. multifiliis* are described in T. Clark et al., Proc. Nat. Acad. Sci. USA, 89:6363-6367 (1992) and T. Clark et al., Gene 229:91-100 (1999); additional exemplary N-terminal and C-tenninal targeting sequences, derived from the 48 kD and 55 kD *I. multifiliis* i-antigens (SEQ ID NOs:5 and 6, Fig. 3(a)), are described in WO 00/46373. The coding sequence of an *I. multifiliis* i-antigen includes two putative signal elements. For example, the N-terminal portions (i.e., about the first 20 amino acids) of the 48 kD i-andgen (SEQ ID NO:5, Fig. 3(a)) and the 55 kD i-antigen (SEQ ID NO:6, Fig. 3(a)) form highly hydrophobic domains which are similar to the signal peptides required in other organisms for translocation of newly translated polypeptides into the endoplasmic reticulum (ER). The C-terminal portions (i.e., about the last 20 to 23 amino acids) of the 48 kD i-antigen (SEQ ID NO:5, Fig. 3(a)) and the 55 kD i-antigen (SEQ ID NO:6, Fig. 3(a)) contain sequences that are homologous to the signals required for addition of a glycosylphosphatidylinositol (GPI) anchor and membrane anchoring of surface-displayed polypeptides. Surprisingly, the targeting signals of the *I. multifiliis* 48 kD i-antigen have been discovered according to the invention to be fully functional in *Tetrahymena,* causing surface display on the *Tetrahymena* plasma membrane of the i-antigen of *I. multifiliis.* Heterologous fusion proteins comprising one or both of these *I. multifiliis* signal sequences are therefore fully expected to be targeted correctly in *Tetrahymena.* For example, the targeting sequences of the *I. multifiliis* i-antigen can be used for surface display in *Tetrahymena* of heterologous surface membrane proteins such as surface antigens or receptors. Moreover, these targeting sequences are also expected to allow surface display or secretion of a polypeptide that is not normally expressed on a cell surface. For example, the protein expression system of the invention can be used for surface display of proteins that may be toxic to the cell when expressed intracellularly, such as proteases that can degrade *Tetrahymena's* own essential cytosolic proteins.

It should be understood that this aspect of the invention involving protein targeting using, for example, N-terminal and C-terminal targeting sequences from *I. multifiliis* and other protozoa applies not just to protein targeting in protozoa but in other cells such as bacterial cells, fungus cells and animal cells including vertebrate cells such as mammalian cells.

Further, other types of targeting sequences are well-known and can be used in the practice of the invention. For example, targeting to the surface of a ciliate can also be achieved if the protein includes a known transmembrane domain, such as the C-terminal 80 amino acids of human membrane cofactor, in place of a GPI anchor (see e.g., J. Seeber et al., J. Cell Sci., 111:23-29 (1998)). In this embodiment, the protein can be partially embedded in the membrane and partially exposed on the outside of the cell.

It should also be understood that recombinant genetic constructs of the invention encode proteins that preferably do, but need not, contain N-terminal and/or C-terminal targeting sequences; a protein without such signaling sequences will simply remain in the cytosol of the host cell.

### Cell surface expression of antigens

The ability to express polypeptides on a protein surface, in particular, gives this system advantages over others known in the art for many purposes. For example, antigens, including haptens, can be expressed on the cell surface as fusion proteins, cleaved, isolated, and injected into laboratory animals to generate antibodies for further use. Alternatively, antigens expressed on the surface of a host cell can be used to identify antibodies directly using a *in vitro* immobilization reaction or chemokinesis of swimming cells. Antisera directed against the i-antigens of hymenostomatid ciliates (including *Paramecium, Tetrahymena* and *Ichthyophthirius* spp.) cross-link cilia and cause rapid immobilization of corresponding cell types in culture (J. Preer, "Surface Antigens of Paratnecium" in J. Gall, ed., Molecular Biology of Ciliated Protozoans, Academic Press, London, 301-339 (1986); F. Caron et al., Ann. Rev. Microbiol. 43:23-42 (1989); Clark et al., Ann. Rev. Fish Dis. 5:113-131 (1995)). For example, an immobilization assay can be conducted on the transgenic cells to detect antibody in serum samples of a parasite-infected patient A positive reaction is detected by immobilization of the live transgenic protozoan cells, due to cross-linking of antibodies bound to surface-displayed parasitic proteins. This method can be extended to detect serum antibodies to other bacterial, parasitic, or fungal infections or diseases, using a live transgenic protozoan genetically engineered in accordance with the invention to display the appropriate antigen on its surface. Likewise transgenic cells of the invention that display antigens on their surface can be used to isolate and purify antibodies. Screening of chemical agents (drugs) for the ability to bind to polypeptides, and purification of drugs thus identified, can also be readily accomplished using the invention, as long as the candidate drugs possess cross-linking capability. Cross-linking capabilities include the provision of multiple potential binding sites on the candidate drugs, or the addition of chemical or radiation-dependent cross-linking sites on the candidate drugs. In the latter case, cross-linking is initiated after binding of the drug to the live ciliate, and the mechanism for cross-linking must be selected such that it does not itself cause immobilization of the transgenic ciliate. For example, the protein expression system could be used for screening multivalent ligands that bind to human neurotransmitter receptors expressed on the surface of transgenic *Tetrahymena* according to the invention.

### Preparative protein production

The invention includes a method for producing a polypeptide that involves introducing into a ciliated protozoan host an expression vector containing a heterologous nucleotide sequence encoding a polypeptide, then expressing the polypeptide. After the vector is introduced into the ciliate host, the host is cultured under conditions that allow expression of the coding region such that the polypeptide encoded by the coding region is produced. The polypeptide can be present within the host cell, secreted from the host cell or is anchored to the surface of the host cell. Optionally, the method further includes isolating the polypeptide. The amino acid sequence of the heterologous polypeptide encoded by the coding region of the vector used to transform the ciliate host optionally includes protein targeting sequences. The polypeptide produced by the transformed host preferably includes either an amino terminal signal peptide or a GPI cleavage/attachment site or both. More preferably, the polypeptide includes both an amino terminal signal peptide and a GPI cleavage/attachment site.

### Transgenic ciliate

The present invention also provides a ciliated protozoan, preferably a nonpathogenic ciliated protozoan, more preferably *Tetrahymena,* that has been genetically engineered to express a heterologous polypeptide, preferably on its cell surface. A surface-expressed heterologous protein is attached by way of a "GPI anchor" that is encoded by a GPI cleavage/attachment sequence. Preferably, the ciliate contains a vector containing a coding region that encodes the heterologous protein, wherein the coding region is integrated into the *BTU1* gene of *T. thermophila.*

### Live and killed vaccines

Success in expressing polypeptides on the surface of a ciliated protozoan has immediate implications for vaccine development. Surface antigens are important targets of the humoral immune response against a wide range of microbial pathogens. Accordingly, the present invention provides a system for vaccination that makes use of surface expression of heterologous antigenic polypeptides encoded by heterologous nucleic acid fragments in a ciliated protozoan.

A vaccine effective for the prevention of infection in an organism is one that elicits the production of a protective immune response in an organism exposed to the vaccine. The immune response can be a cellular response and/or involve the production of antibodies. The goal of vaccination is to elicit a population of lymphocytes, which upon subsequent exposure to the disease causing agent, such as a parasite, proliferate and produce antibodies and/or effector cells specific to the parasite, resulting in protection against lethal infections.

The vaccine of the invention can be either a live vaccine or a killed vaccine. It is useful to prevent disease in vertebrates, including humans, dogs, cats, reptiles, poultry, cattle, swine and fish. Preferably, the vaccine of the invention is effective to prevent disease in fish, more preferably it is effective to prevent parasitic infection in fish. The vaccine preferably comprises a live or killed host ciliated protozoan having, attached to its plasma membrane and displayed on it surface, a heterologous antigenic polypeptide. The heterologous protein is preferably GPI-anchored to the plasma membrane. In a particularly preferred embodiment of the vaccine, the heterologous antigenic polypeptide that is surface expressed comprises all or an immunogenic portion of an immobilization antigen ("i-antigen") of a pathogenic ciliated protozoan. I-antigens are surface protein that are generally abundant on the surface of a ciliate; an antibody that is specific for an i-antigen causes a loss of protozoan motility, and can cause agglutination of the cilia. Use of a whole cell vaccine, whether live or killed, has the additional advantage of potentially serving as an adjuvant, thus further stimulating the immune system of the recipient.

White spot disease, also called "Ich," is a disease caused by the parasite *I. multifiliis,* a holotrichous ciliated protozoan which is an obligate parasite of freshwater fish. Under conditions of intensive aquaculture, Ich frequently has a high morbidity and mortality, resulting in significant financial losses to fish producers. Attempts to control of prevent *I. multifiliis* infections have met with only limited success. Killed *I. multifiliis* cells and *I. multifiliis* cilia alone do not elicit protective immunity. Nor has vaccination of fish with the cells or cilia of other ciliates, such as *T. thermophila* cells or cilia, generated protective immunity against *Ich* infection (Gratzek et al., U.S. Pat. No. 4,309,416). Chemical treatments are likewise unsatisfactory because carcinogenicity and/or toxicity are typically associated with these therapeutic agents. See Burkart et al., J. Fish Dis., 13:401-410 (1990), for a summary. Moreover, *I. multifiliis* can only be grown in association with its host, and efforts to produce i-antigens in conventional protein expression systems are inconvenient at best.

Significantly, *Tetrahymena* is nonpathogenic and is normally present in freshwater ecosystems. It thus represents an ideal vehicle for vaccination against *I. multifiliis* infection and other fish diseases. The use of *T. thermophila* for the expression and delivery of the antigens will make possible large-scale, cost-effective fish vaccination programs.

A vaccine against *I. multifiliis* infection preferably comprises live or killed *T. thermophila* that have been engineered according to the invention to express all or an immunogenic portion of an i-antigen from *I. multifiliis* on its surface. Preferably, the i-antigen used in the live or killed *T. thermophila* vaccine of the invention is encoded by i-antigen encoding sequences of either *IAG48[G1]* or *IAG55[G5]* (Fig. 3(b)). In a particularly preferred embodiment, the vaccine comprises transgenic *T. thermophila* wherein expression of the *I. multifiliis* i-antigen is achieved by insertion of the i-antigen into the endogenous *btu1-1K350M* locus. Because this transformant can be maintained by selection, a selectable drug-resistance gene does not need to be introduced into the transgenic host, eliminating the possible introduction of antimicrobial resistance genes into the environment upon release of transgenic *Tetrahymena.*

In the ciliated protozoan genetic code, TAA and TAG encode glutamine, whereas they serve as stop codons in the "universal" genetic code. The presence of glutamine-encoding TAA and TAG codons in the native coding sequence of the i-antigen creates barriers to expression of this sequence in conventional bacterial gene expression systems. Advantageously, *T. thermophila* recognizes UAA and UAG codons as glutamine codons as does *I. multifiliis.* In addition, post-translational modifications, particularly glycosylation, are expected to occur more normally in organisms related to *Ichthyophthirius* (for example, *Tetrahymena*) than in prokaryotes such as *E*. *coli,* or more distantly related eukaryotes. Because such modifications can play a critical role in immune recognition, *Tetrahymena* is particularly advantageous in accordance with the present invention.

As shown in the following Examples describing the surface localization of *Ichthyophthirius* proteins in *Tetrahymena IAG48[G1]* transformants, the N-terminal and C-terminal protein targeting peptides associated with *I. multifiliis i-*antigen function appropriately in *Tetrahymena,* i.e., the antigenic protein is expressed on the surface of transgenic *Tetrahymena.* Moreover, the overall distribution of *I. multifiliis* antigens in transformed *Tetrahymena* cells bear a striking resemblance to the pattern seen in the parasite itself where they are found in ciliary and plasma membrane, and in the cell cortex in association with secretory mucocysts.

The invention further can be used in a method for vaccinating an animal that includes contacting the animal with a genetically engineered nonpathogenic ciliated protozoan, preferably *Tetrahymena,* so as to elicit a protective immune response in the animal to the heterologous antigen displayed on the surface of the protozoan host. For example, an animal can be exposed to an ingestable composition, such as food, water, or both, that contains an immunogenic genetically engineered nonpathogenic ciliate of the invention. Alternatively, an animal can be immersed in or sprayed with an aqueous solution that includes the immunogenic ciliate. For example, fish can be vaccinated in accordance with the invention by exposing the fish to a composition including transgenic *T. thermophila* expressing an antigenic polypeptide (e.g., the *I. multifiliis* i-antigen to vaccinate against white spot disease). The fish can be immersed in an aqueous solution comprising transgenic *T. thermophila* expressing the *I. multifiliis* i-antigen. Because the antigenic proteins are presented to the immune system, probably through the lateral line and gills, the fish can begin to produce protective antibodies to at least a portion of the *I. multifiliis* i-antigen.

The vaccine can also be administered by injecting the animal with live or killed genetically engineered nonpathogenic immunogenic ciliated protozoa, by injecting the animal with a purified or partially purified membrane fraction of cells of the genetically engineered nonpathogenic immunogenic ciliated protozoa, or by injecting the animal with cilia from cells of the genetically engineered nonpathogenic immunogenic ciliated protozoa. Additionally, genetically engineered protozoa that display an antigen on the surface according to the present invention can also be used to generate monoclonal antibodies directed to the surface-expressed polypeptide.

### EXAMPLES

The following examples are provided for illustrative purposes only and are not intended to limit the scope of the invention.

The examples utilize many techniques well-known and accessible to those skilled in the arts of molecular biology, and in the transformation of *Tetrahymena.* Enzymes are obtained from commercial sources and are used according to the vendors' recommendations or other variations known in the art. Reagents, buffers and culture conditions are also known to the art. References containing standard molecular biological procedures include Sambrook et al. (1989) Molecular Cloning, Second Edition, Cold Spring Harbor Laboratory Press, Plainview, New York; Maniatis et al. (1982) Molecular Cloning, Cold Spring Harbor Laboratory, Cold Spring Harbor, New York; Wu (ed.) (1979) Methods Enzymol. 68; Wu et al. (eds.) (1983) Methods Enzymol. 100 and 101; Grossman and Moldave (eds.) (1980) Methods Enzymol. 65; Miller (ed.) (1972) Experiments in Molecular Genetics, Cold Spring Harbor Laboratory, Cold Spring Harbor, New York; Old and Primrose (1981); Principles of Gene Manipulation, University of California Press, Berkeley, California; Schlief and Wensink (1982) Practical Methods in Molecular Biology; Glover (ed.) (1985) "DNA Cloning", Vols. I and II, IRL Press, Oxford, UK; Hames and Higgins (eds.) (1985) Nucleic Acid Hybridization, IRL Press, Oxford, UK; Setlow and Hollaender (1979) Genetic Engineering: Principles and Methods, Vols. 1-4, Plenum Press, New York.

References containing molecular biological techniques, procedures and protocols that are particularly useful in ciliated protozoa include J. Boothroyd et al. (eds.) Molecular Approaches to Parasitology, John Wiley & Sons, New York and J. Gall (ed.) (1986) The Molecular Biology of Ciliated Protozoa, Academic Press, Inc., Orlando, FL.

Abbreviations and nomenclature, where employed, are deemed standard in the field and commonly used in professional journals such as those cited herein.

### Example I. Plasmid Construction: pBAB1 and pICH3

The p*BTU1* plasmid containing a 3.7 kb Bgl II/Hind III fragment of the *Tetrahymena thermophila* β-tubulin gene, *BTU1,* was used to construct a derivative, pBAB1, in which the entire coding sequence was replaced with the coding sequence of the neomycin resistance gene, *neo1* (R. Kahn et al., Proc. Nat'l. Acad. Sci. USA 90:9295-9299 (1993)). Thus, the expression of *neo1* gene on pBAB1 is driven by the flanking sequences of the *BTU1* gene. The plasmid pBAB1 was used to construct another derivative in which the *neo* coding region was replaced with the entire coding sequence of the *Ichthyophthirius* i-antigen (isolate G1) pre-protein. T. G. Clark et al., Proc. Natl. Acad. Sci. USA, 89: 6363-6367 (1992). To this end, the pBAB1 plasmid was amplified with the *NEO257* primer (5'-AGCCAGTCCCITCCCGCITCAGTGACAA-3' (SEQ ID NO:9) (provided by J. Bowen and M. Gorovsky, University of Rochester, NY) whose sequence encodes the antisense strand of the *neo1* gene, and primer BTN3 (5'-CGGGATCCAGCGAACTGAATCGGTCAGCT-3') (SEQ ID NO:10), corresponding to the 3' noncoding region of *BTU1* located immediately downstream from the stop codon, TGA, and running in the same direction as the sense coding strand. The BTN3 primer contains a BamH I restriction site sequence at its 5' end, positioned immediately downstream from the stop codon, TGA. The product of PCR amplification of pBAB1 using *NEO257* and BTN3 primers contained the noncoding sequences of *BTU1,* an N-terminal half of the *neo1* gene coding region and the vector sequence. A single Hind III site is located almost immediately downstream from the translation initiation codon, ATG, of the *neo1* gene. The amplified product was digested with BamH I and Hind III to obtain a smaller fragment containing the entire flanking sequences of *BTU1* and the plasmid vector. A plasmid carrying a 2 kb EcoR I genomic fragment of the *Ichthyophthirius* i-antigen gene was amplified with primers IC5 (5'CCCAGCTTGAAATATAATATTI'TATTAATTTTAATT-3') (SEQ ID NO:11) and IC3 (5'-AGGGATCCTCACAATAAATAGAAAGAAATAA3') (SEQ ID NO:12). These primers amplified the entire coding sequence of the *Ich* i-antigen gene and introduced Hind III, and BamH I restriction sites at positions encoding the N-terminal and C-terminal end of the protein respectively and corresponding to the positions of the same restriction sites present on the amplified fragment of pBAB1. This PCR product was digested with Hind III and BamH I, and ligated to the BamH 1-Hind III digest of amplified pBAB 1 to give pBICH3 plasmid, which contains the entire coding sequence of the i-antigen gene inserted in a correct translational frame between the regulatory sequences of the *BTU1* gene (see Figure 1). This new hybrid gene was designated *BTU1-4::IAG48[G1].*

### Example II. DNA-mediated Transformation of T. thermophila

### Transformation

A *Tetrahymena thermophila* strain, CU522 (provided by Dr. Peter Bruns, Cornell University, Ithaca NY) was used as a transformation host. This strain carries a single substitution (K350M) in the β-tubulin, *BTU1* gene. This mutation was originally described for *Chlamydomonas reinhardtii* (Bolduc et al., Proc. Nat'1. Acad. Sci. USA 85:131-135 (1988)), and was later found to confer a similar phenotype in *T. thermophila,* namely, increased resistance to several microtubule depolymerizing drugs, such as oryzalin and colchicine, and increased sensitivity to a microtubule stabilizing agent, paclitaxel (J. Gaertig et al., Proc. Nat'l. Acad. Sci. USA 91:4549-4553 (1994)).

Cells were grown in 50 ml of SPP medium (J. Gaertig, Proc. Nat'l. Acad. Sci. USA, 91:4549-4553 (1994)), supplemented with 100 U/ml penicillin, 100 µg/ml streptomycin and 0.25 µg/ml amphotericin B (SPPA medium), in 250 ml Erlenmayer flasks with shaking at 150 rpm at 30°C. Prior to transformation, the CU522 cells were grown to a density of about 6 x 10⁵ cells/ml in SPPA with shaking at 30°C. Twenty-four hrs before transformation, 50 ml of growing cells were washed and suspended in 10 mM Tris-HCl (pH 7.5) buffer in the original volume. After 4-5 hrs starving cells were counted again, cell concentration adjusted to 3 x 10⁵ cells/ml and cells left at 30°C without shaking for another 18-20 hrs.

To target the *BTU1* gene derivatives to the endogenous *BTU1* locus of *T. thermophila,* either pBAB or pBICH3 plasmids were digested with Sac I and Sal I restriction endonucleases to separate the insert (either *BTU1-2::neol* or *BTU1-4::IAG48[G1]*) from the plasmid. Fifty micrograms of digested DNA was purified by a single phenol/chloroform/isoamyl alcohol (25:24:1) extraction followed by chloroform/isoamyl alcohol (24:1) extraction, precipitated with an equal volume of isopropyl alcohol in the presence of 0.15 M sodium acetate, dried and resuspended at 1 mg/ml.

The transformation method used in this study targets genes into the somatic macronucleus of vegetative cells using biolistic bombardment (D. Cassidy-Hanley et al., Genetics 146:135-147 (1997)). Ten micrograms of linearized plasmid DNA was used to coat 60 mg of 0.6 µm gold particles (Bio-Rad) using the Sanford Large Batch DNA Coating Method, as described by J.C. Sanford et al., Bio Techniques, 3:3-16 (1991). An aliquot of 2.4 x 10⁷ of starved cells was spun down at 600 x g for 3 min, washed with 45 ml of 10 mM Tris (pH 7.5) and resuspended in 3 ml of Tris buffer. One ml of cells was bombarded using 10 µg of DNA-coated gold particles at 900 psi using DuPont Biolistic PDS-1000/He particle delivery system (Biorad). Bombarded cells were resuspended in 50 ml of SPPA, left for 2-3 hours at 30°C. Paclitaxel was added to the final concentration of 20 µM and cells were plated on microtiter plates using 100 µl cells per well and plates were incubated in moist boxes at 30°C (in darkness, to prevent photobreakdown of paclitaxel). Wells containing paclitaxel-resistant transformants were apparent after 2-3 days of selection.

To identify sequences integrated into the *BTU1* locus of *T. thermophila,* total genomic DNA was isolated from transformants using the fast urea method as described by J. Gaertig (Proc. Nat'l. Acad. Sci. USA, 91:4549-4553 (1994)) and used as a template for PCR with primers: *BTU1*-75 (5*'-*AAAAAATAAAAGTTTGAAAAAAA-3') (SEQ ID NO:13), sequence located 53-75 bp upstream to the ATG translation initiation codon in the *BTU1* 5' flanking region, and primer *BTU1-*3, (5'-GTTTAGCTGACCGATTCAGTTCG-3') (SEQ ID NO:14), located close to the TGA stop codon in the 3' flanking region of *BTU1.* The resulting amplified products were digested with Nsi I restriction endonuclease and run on a TAE-agarose 0.7% gel.

### Immunocytochemistry

For immunofluorescent detection of the *I. multifiliis* i-antigen in transgenic *Tetrahymena* cells, 10 mls of exponentially growing cultures (2 x 10⁵ cells/ml) were harvested, washed with 10 mls of 10 mM Tris (pH 7.5) and resuspended in 0.5 ml. Cells were fixed with 3.5 mls of 2% paraformaldehyde in PHEM buffer (60 mM Pipes, 25 mM HEPES, 10 mM EGTA, 2 mM MgCl₂, pH. 6.9) for 30 minutes at room temperature. Fixed cells were washed once with 3 mls of PHEM buffer and 2 times with 3 mls of modified phosphate buffered saline, PBS (130 mM NaCl, 2 mM KCl, 8 mM Na₂HPO₄, 2 mM KH₂PO4, 10 mM EGTA, 2 mM MgCl₂, pH. 7.2) and resuspended in 0.5 ml. For antibody labeling, 100 µl of fixed cells were washed 3 times for 10 minutes with 3 mls of PBS-BT, (3% bovine serum albumin, 0.1% Tween 20 in PBS), and concentrated in 100 µl. One µl of affinity-purified rabbit antiserum directed against *Ichthyophthirius* G1 i-antigen (T.L. Lin et al., J. Protozool., 39:457-463 (1992)), was added, followed by incubation overnight at 4°C, three washes in PBS-BT and incubation with the detection antibodies (at 1:100 dilution) for 45 minutes at room temperature.

Secondary antibodies were goat anti-rabbit IgG coupled to FITC (Zymed). Labeled cells were washed 3 times with PBS, concentrated in 0.1 ml of PBS plus 10 µl of DABCO mounting medium (100 mg/mll,4-diazobicyclo-[2,2,2]-octane, Sigma Chemical Co., dissolved in 90% glycerol in PBS). To mount, 5 µl of cells were combined with 5 µl of DABCO medium, covered with a cover-slip and sealed with nail polish. Slides were examined with a Bio-Rad MRC 600 Laser Scanning Confocal Microscope at the UGA Center for Advanced Ultrastructural Research. Sets of optical sections of individual cells were processed to obtain complete 3-D reconstructions.

### Immunoblotting

Protein extracts were prepared from 1-2x10⁶ cells taken from exponentially growing cultures. Cells were spun down at 2000 rpm for 5 minutes, washed with 10 mls of ice-cold 10 mM Tris, pH 7.5, and resuspended in 125 µl of ice-cold Tris buffer supplemented with a mixture of protease inhibitors which included 0.5 µg/ml leupeptin, 10 µg/ml E-64, 10 µg/ml chymostatin and 12.5 µg/ml antipain (A. Turkewitz , personal communication, all inhibitors from Sigma), combined with 125 µl of boiling 2xSDS-PAGE sample buffer and boiled for 5-10 min. Ten µl of extracts were loaded on a 10% SDS-PAGE minigel and proteins transferred on nitrocellulose using semi-dry transfer system (Biorad). The filter was blocked for 2 hr in PBS-T buffer containing 5% dried milk, followed by incubation with the anti-*I. multifiliis* i-antigen antibodies (1:10000) overnight at 4°C. The filter was washed extensively with PBS with 0.1 % Tween-20 (PBST) and incubated in the same buffer containing the goat-anti-rabbit IgG antibodies conjugated to alkaline phosphatase (Bio-Rad) for 1 hr at room temperature. The membrane was washed in PBST and developed using NBT/BCIP (Bio-Rad) as described by M. Gorovsky, J. Protozool., 20:19-25 (1973)

### Immobilization Assay

Growing *Tetrahymena* cells were washed with 10 mM Tris, pH 7.5 and resuspended at 2000 cells/ml. Cells were incubated with a series of dilutions of monospecific rabbit polyclonal antiserum against the parasite i-antigen 48 kD protein on microtiter plates at 1000 cells/ml in 100 microliter/well aliquots. After 15-60 minutes wells were scored under a dissecting scope. The behaviors of both *BTU1-4::IAG48[G1]* transformants and controls (*neol* transformants) were examined, and the percentage of immobilized cells was estimated.

### Results

Cells were selected for resistance to either paclitaxel (30 µM) or paromomycin (120 µg/ml). As shown in Table I, transformants were readily obtained following selection with either drug, but were not detected in a mock transformation experiment. Furthermore, random clones that had been selected for growth in paclitaxel were all found to be cross-resistant to paromomycin (n=40), and nearly all transformants originally selected with paromomycin, produced clones resistant to 30 µM paclitaxel (89%, n=71). Thus, acquisition of the transformed phenotype (in most, if not all cases) resulted from disruption of the host *btu1-1K350M* gene, and negative paclitaxel selection based on *BTU1* gene loss-of-function was nearly as effective as a positive selection based on paromomycin resistance conferred by the transgene.

**Table I. Transformation of T. thermophila using biolistic bombardment**

| Targeting Fragment | Selection method | Frequency (transf./µg DNA) |
|---|---|---|
| *BTU1-2::neo1* | tx* | 115 |
| *BTU1-2::neo1* | pm^{¶} | 204 |
| No DNA | tx | 0 |
| No DNA | pm | 0 |
| | | |
| *BTU1-2::neol* | tx | 37 |
| *BTU1-4::LAG[G1]* | tx | 31 |
| No DNA | tx | 0 |

| | | |
|---|---|---|
| *30 µM paclitaxel. ^{¶}120 µg/ml paromomycin. | | |

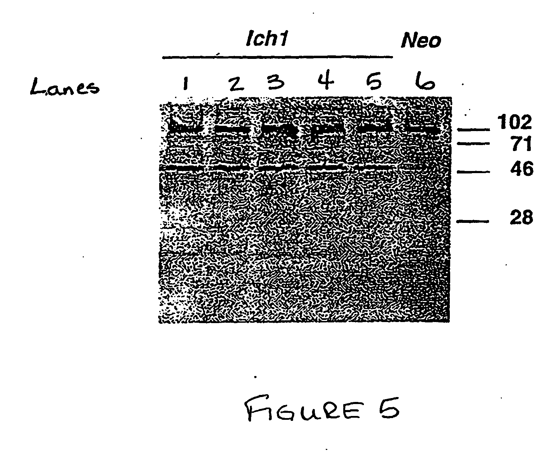

Unlike the bacterial *neo* gene, the product of the i-antigen gene of *I. multifiliis* is not inherently selectable. Nonetheless, as shown in Table I, paclitaxel-resistant transformants were obtained with a frequency comparable to that seen with the control *BTU1-2::neol* gene construct. Western blotting studies (Fig. 5) using polyclonal antiserum against affinity purified i-antigens of *Ichthyophthirius* showed that a protein of apparent MW of 50 kDa was detected in all transformants carrying the *IAG48[G1]* gene (lanes 1-5), but not in a control cell line transformed with *neol* (lane 6). The size of the detected protein was in close agreement with the MW of the corresponding surface antigen of *Ichthyophthirius.* An additional band of about 100 kDa seen in both *BTU1-4::IAG48[G1]* and control extracts, most likely represents an endogenous *Tetrahymena* protein that shares immunological determinants with the parasite antigen.

It should be noted that because the macronucleus of *T. thermophila* is polyploid and contains about 45 copies of each chromosome, only partial replacement of endogenous genes occurs following initial transformation. Nevertheless, chromosomes are distributed to daughter nuclei through an imprecise form of allelic segregation known as amitosis, and complete replacement of endogenous genes by transgenic copies can be achieved by continuous growth in selective media. In this case, PCR analysis revealed that about one half of endogenous copies of the *BTU1* gene were replaced by the *IAG48[G1]* transgene during early stages of selection. Following growth in the presence of increasing concentrations of paclitaxel, however, endogenous copies of the *BTU1* gene could no longer be detected, and a corresponding increase in the copy number of *IAG48[G1]* was seen, indicating that they had lost all copies of the endogenous *BTU1* allele.

In *Ichthyophihirius,* the 48-kDa protein is a major component of the cell surface and is bound to ciliary and plasma membranes through a glycosylphosphatidylinositol (GPI) anchor. Nevertheless, the sequence elements responsible for membrane targeting in ciliates are not well-understood, and while *Ichthyophthirius* and *Tetrahymena* are taxonomically related, it was not know whether signal peptides from such widely diverged species could function interchangeably.

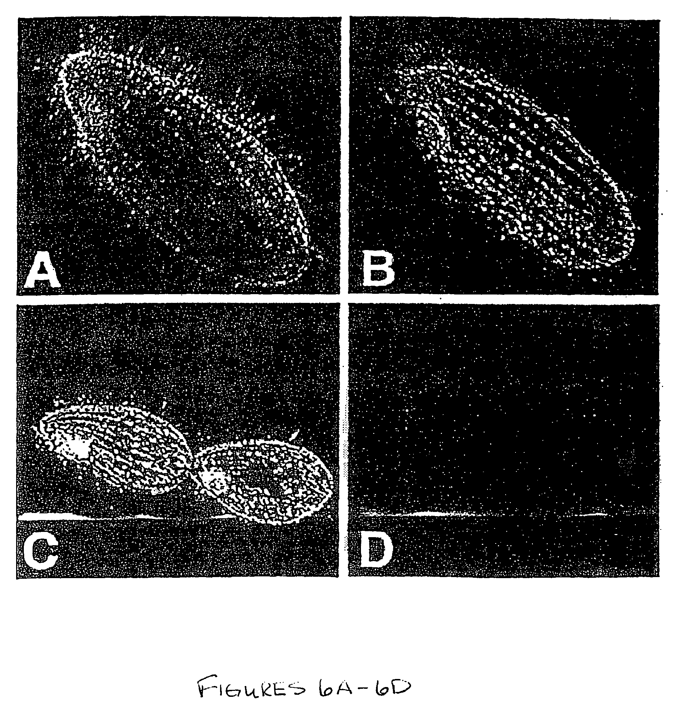

Transformed cell lines were therefore fixed (without permeabilization), reacted with antibodies against the 48-kDa antigen and analyzed for the presence of the *I. multifiliis* protein on their surface by indirect immunofluorescence using confocal microscopy. Strong labeling of oral and somatic cilia was observed (Fig. 6A, 6B and 6C), wherein somatic cilia are shown in Fig. 6A and oral cilia are shown in Fig. 6C. Surface cortical fluorescence was seen in the form of longitudinal rows (shown in Fig. 6B). Dividing cells showed labeling in both the preexisting and newly formed oral apparatus, indicating that as the new structure is formed, parasite antigens are rapidly inserted into the cell membrane (Figure 6C). Control cells (transformed with *neo1*) showed no obvious labeling (Figure 6D). Fluorescence was only seen in controls when cells were permeabilized with methanol prior to fixation with paraformaldehyde. This appeared as a generalized background staining, and was not surprising given the presence of a cross-reacting (100 kDa) band in Western blots of control cell extracts.

A further demonstration of the presence of *I. multifiliis* proteins on the surface of *Tetrahymena* came from immobilization assays with living cells. In the immobilization assay, at antisera dilutions of 1:100 or less, rabbit antisera against the 48-kDa protein caused an immediate reduction in swimming velocity of the *BTU1-4::IAG48[G1]* transformants, followed by a complete loss of motility within 50 minutes. In contrast, *neo1* transformants showed no immediate effect, but eventually became immobilized after 90 minutes. The difference in the response of *BTU1-4::IAG48[G1]* versus *neo1* transformants indicates that *I. multifiliis* antigens are accessible to antibodies at the surface of live cells. Furthermore, because immobilizing antibodies recognize 3-dimensional epitopes on the 48 kDa protein, the antigen itself is likely to assume a native conformation in *Tetrahymena.*

### Example III. Expression of Green Fluorescent Protein in Tetrahymena

Using the *Tetrahymena BTU1* targeting system (Examples I and II), a modified version of the G 1 i-antigen was constructed in which the green fluorescent protein (GFP) tag coding sequence was inserted in a correct frame into the G 1 i-antigen of *I*. *multifiliis,* about 10 amino acids downstream from the site of cleavage of the N-terminal signal peptide. The G1-GFP construct was thereby targeted to the *BTU1* locus, and transformants were selected with paclitaxel. The fusion protein was detected inside transformed cells using Western blotting and anti-gfp antibodies. A fraction of the fusion protein was found to be associated with the Triton X-114 extractable membranes of transformant cells, although no fusion protein was found on the cell surface.

### Example IV. Expression of Malaria Surface Protein in Tetrahymena

Malaria is caused by parasitic protozoa of the genus *Plasmodium,* and is responsible for 2 million deaths a year worldwide. Current control efforts are focused on the mosquito vector, and treatment of the disease with anti-malarial drugs. No vaccine is yet available. Over the past four decades the parasite has become resistant to many anti-malarial drugs, and an effective vaccine is seen as best hope for controlling morbidity and mortality caused by this pathogen.

Most of the candidate molecules from *Plasmodium* suggested for inclusion in a vaccine are proteins that bind to receptors on host target cells. Necessary for vaccine development is the identification and characterization of the role played by these proteins. Unfortunately, this effort has been hampered by difficulties in procuring enough parasite protein to allow thorough study. Several heterologous expression systems have been utilized, but none has proven to be ideal, especially for the production of functional protein.

In this preliminary study, the *Tetrahymena BTU1* targeting system (Examples I and II) was evaluated for use in expression of the *Plasmodium falciparum* surface proteins implicated in interactions with the host cells. A 1,900 bp region of a putative *P. falciparum* erythrocyte binding protein, EBL-1, was directly inserted in frame, at a *NsiI* restriction site, into the coding sequence of the G1 i-antigen of *I. multifiliis* flanked by the *BTU1* targeting sequences. The resulting construct had a coding region encoding a chimeric fusion protein having the N-terniinal portion of the i-antigen (representing about 2/3 of the i-antigen protein), followed by the EBL-1 domain encoded by the 1,900 bp region, followed by the C-terminal portion of the i-antigen (representing about 1/3 of the i-antigen protein), under control of the *Tetrahymena BTU1* promoter.

This construct was expressed in *Tetrahymena,* demonstrating that the presence within the *P. falciparum* coding sequence of codons that are very rare in Tetrahymena did not abrogate expression. However, this EBL1::G1 fusion protein was expressed in *Tetrahymena* as a cytosolic protein, and no association with the cell membrane or the cell surface was observed despite the fact that the N-terminal and C-terminal targeting sequences of G1 i-antigen were present in the fusion protein. This result could be explained by improper folding of the multi-domain chimeric fusion protein; insertion of the EBL-1 coding sequence into the middle of the G1 i-antigen coding sequence arbitrarily divided the i-antigen sequence into two portions, possibly disrupting folding of the protein or otherwise interfering with the production of a protein that was competent to translocate into or across a membrane. A second experiment replacing the i-antigen coding sequence (between the two targeting sequences) with the EBL-1 domain instead of simply inserting it within the i-antigen coding sequence will provide more insight into targeted expression of EBL-1 in *Tetrahymena.*

### Example V. Deletion of GPI Anchor Modification Domain

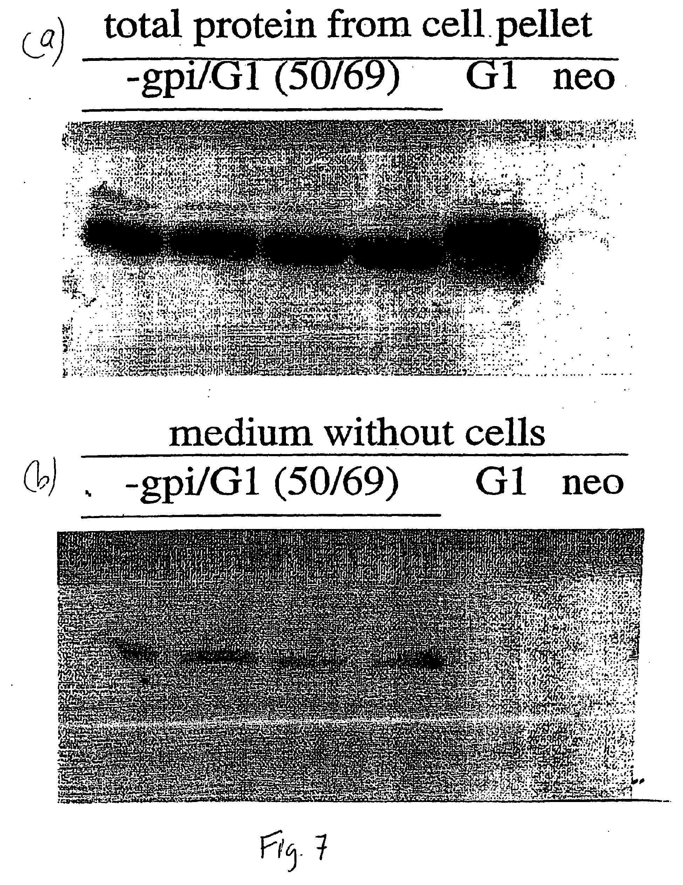

A truncated version of the G1 i-antigen gene from *I. multifiliis* was prepared in which the C-terminal domain implicated in the GPI anchor addition was deleted. The truncated gene was expressed in *Tetrahymena* as in Example II. Figure 7 shows Western blots of total protein extracts of transformant cells (Fig. 7(a)) and total culture medium from which transformant cells were removed (Fig. 7(b)). The first four lanes from the left contained either cells (Fig. 7(a)) or culture medium (Fig. 7(b)) of four independent transformants of *T. thermophila* in which a truncated version of the G1 antigen lacking the GPI-anchoring C-terminal domain was incorporated into the *BTU1* locus. Controls contained either the *neo* gene or a full-length G1 i-antigen of *I. multifiliis* expressed in the *BTU1* locus. The amount of culture medium loaded (Fig. 7(b)) corresponds to about 1/30 of the amount of cells loaded (Fig. 7(a)). The truncated protein was expressed at essentially the same level observed for surface-targeted expression of the gene (Example II) (Figure 7, top). However, about ½ of total expressed truncated G 1 i-antigen protein was found in the culture medium, in contrast to the full length G1 i-antigen protein which was not detected in the culture medium. Thus, the elimination of the GPI anchor domain caused the i-antigen to be secreted out of the cell, as expected.

### Example VI. Expression of Chicken Ovalbumin in Tetrahymena

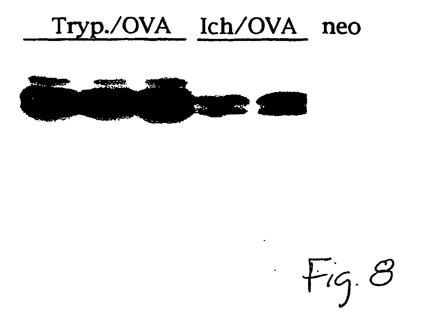

To test the feasibility of *Tetrahymena thermophila* for expression of vertebrate proteins, we prepared two constructs in which the region of the coding sequence of chicken ovalbumin protein (amino acids 139-385; GenBank Acc. Numbers M34346 and M25173; J. Catterall et al., Nature 275:510-513 (1978)) was fused to an N-terminal signal peptide of either the gp-72 surface protein of *T. cruzi* (Tryp./OVA) or of the G1 antigen of *I. multifiliis* (Ich/OVA) subcloned between the flanking sequences of the *BTU1* gene of *T. thermophila.* These constructs were integrated into the *BTU1* locus using biolistic transformation of the paclitaxel-sensitive host cells, and protein extracts of several paclitaxel-resistant transformants were analyzed by a Western blot using anti-chicken ovalbumin antibodies (Sigma Chemical Co.). Two protein bands close to the expected molecular weight of about 30 kD were detected in transformants but not in the negative control cells in which the *BTU1* locus was transformed using the bacterial neomycin resistance gene (*neo*) (Fig. 8). The presence of two bands may be the result of posttranslational modifications or proteolysis in *Tetrahymena.* Interestingly, the fusion protein containing the signal peptide of *T. cruzi* gp72 protein was expressed at several fold higher level compared to the fusion protein with the *I. multifiliis* signal peptide. Also, it is striking that the levels of expression are extremely reproducible among individual transformants.

### Example VII. Immune Response of Channel Catfish to Live Vaccine: Transformed Tetrahymena Expressing Full-length or Truncated I. multifiliis (Serotype A) 48 kD I-antigen Protein and Heterologous Challenge with I. multifiliis (Serotype D)

The *IAG48[G1]* gene of *Ichthyophthirius multifiliis* G1 encodes the GPI anchored 48- kDa i-antigen. The extreme 3' region of the gene encodes a stretch of 14 mostly hydrophobic amino acids separated by a short spacer from three small amino acids (CAS). This sequence encodes the protein's GPI anchor addition site. *Tetrahymena thermophila* cells transformed with the entire *IAG48[G1]* gene produce an intact i-antigen anchored to the cells' surface. *Tetrahymena* cells transformed with a modified *LAG48[G1]* gene construct lacking the 3' sequence which includes the GPI addition site would be expected to produce a truncated protein lacking the GPI anchor.

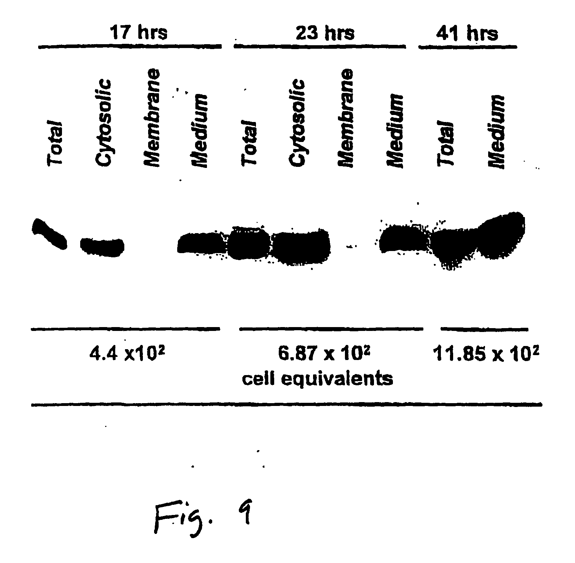

*Tetrahymena* cells were transformed with either the entire *Ichthyophthirius* G1 48- kDa i-antigen protein, or a truncated gene sequence which encodes the i-antigen protein lacking 19 amino acids at the carboxy terminus. Transformants encoding the intact or C-terminal truncated i-antigen were grown in standard *Tetrahymena* growth medium. Cell pellets and supernatant fluids were collected at the time points indicated. I-antigen was detected in cell cytosol, cell membrane or cell culture supernatants by Western blots using rabbit antisera against affinity purified *Ichthyophthirius* G5 i-antigen (see Fig. 9). It is clearly seen that the truncated protein is secreted into the culture medium.

Groups of channel catfish (6 fish per group) were immunized by bath exposure (10⁶ or 10⁵ cells/fish) or intraperitoneal injection with *T. thermophila* transformants (10⁶, 10⁵, or 10⁴ cells/fish) producing intact or truncated i-antigen. A third group of fish was immunized with membrane protein extracts (1 mg or 0.1 mg/fish) from *T. thermophila* producing the full length protein.

*Immunization by bath exposure to Tetrahymena transformants.* Two groups of fish (6 fish in each group) were immunized by bath exposure. The fish were exposed to either 10⁶ or 10⁵ cells /fish for a period of 24 hours. Two immunogens were used: 1) transformed *Tetrahymena* cells expressing the entire *Ichthyophthirius* G1 48-kDa protein, and 2) transformed cells secreting a truncated form of the i-antigen lacking the GPI anchor. Fish in the control group were exposed to *Tetrahymena* transformants expressing the *neol* gene product. Fish were exposed twice at a 30 day interval and challenged 30-60 days after the last immunization with the G5 *Ichthyophthirius* isolate. There were no significant differences (z test) between test and control groups (see Table 1). Immunized fish were challenged with a heterologous strain *of Ichthyophthirius* expressing a different i-antigen (serotype D; G5 isolate) than that produced by the recombinant *Tetrahymena* used for vaccination. It is expected that challenge with a strain *of Ichthyophthirius* producing an i-antigen homologous to the G1 48 i-antigen would show increased levels of protection.

**TABLE 1. Vaccination by bath exposure**

| Immunogen | Dose (cells/fish) | Number of fish challenged | Number of fish surviving | % survival | RSP³ | MDD⁴ ± SD⁵ |
|---|---|---|---|---|---|---|
| Neo control | 10⁶ | 5 | 2 | 40 | N.A. | 17.0 ± 1.0 |
| TG1¹ | 10⁵ | 5 | 3 | 60 | 33.3 | 18.5 ± 2.1 |
| TG1 | 10⁶ | 6 | 3 | 50 | 25.0 | 13.3 ± 4.9 |
| sTG1² | 10⁵ | 6 | 3 | 50 | 25.0 | 21.0 ± 4.6 |
| sTG1 | 10⁶ | 6 | 4 | 66.7 | 40.0 | 17.0 ± 1.4 |

| | | | | | | |
|---|---|---|---|---|---|---|
| ¹ *Tetrahymena* expressing intact membrane form of *Ichthyophthirius* G1 i-antigen. ² *Tetrahymena* secreting truncated form of G1 i-antigen. ³ Relative Survival Percent =1- (number of dead fish in test group/number of dead fish in control group) x 100% ⁴ Mean days to death ⁵ Standard deviation | | | | | | |

*Immunization by injection of Tetrahymena transformants.* Fish in each group were injected intraperitoneally with 10⁶, 10⁵, or 10⁴ live transformed *Tetrahymena* cells/fish. The same immunogens and controls were tested as in the immersion vaccinations. Fish were injected two times at a 30 day interval, and challenged 21 days after the last immunization with G5 *Ichthyophthirius.* A greater degree of protection was elicited in immunized fish compared to controls (Table 2).

**TABLE 2. Vaccination by injection**

| Immunogen | Dose (cells/fish) | Number of fish challenged | Number of fish surviving | % survival | RSP³ | MDD⁴ ± SD⁵ |
|---|---|---|---|---|---|---|
| Neo control | 10⁵ | 6 | 2 | 33.3 | N.A. | 15.3 ± 3.6 |
| TG1¹ | 10⁶ | 5 | 3 | 60 | 44.5 | 19.0 ± 2.8 |
| TG1 | 10⁵ | 5 | 4 | 80 | 58.4 | 15.0 ± 0.0 |
| TG1 | 10⁴ | 6 | 2 | 33.3 | 0 | 14.0 ± 1.4 |
| sTG1² | 10⁶ | 6 | 5 | 83.3 | 50.0 | 21.0 ± 4.6 |
| sTG1 | 10⁵ | 6 | 3 | 50.0 | 25.0 | 20.0 ± 5.7 |

| | | | | | | |
|---|---|---|---|---|---|---|
| ¹ *Tetrahymena* expressing intact membrane form *of Ichthyophthirius* G 1 i-antigen. *² Tetrahymena* secreting truncated form of G1i-antigen. ³ Relative Survival Percent = 1- (number of dead fish in test group/number of dead fish in control group) x 100% ⁴ Mean days to death ⁵ Standard deviation | | | | | | |

*Serum antibody production.* Fish serum antibody responses against recombinant G1 *Ichthyophthirius* i-antigen were determined by ELISA at 2, 4, and 6 weeks after immunization. Serum antibodies from immunized fish were detected with a sandwich ELISA technique that used wells coated with a cross-reactive rabbit antibody against *Ichthyophthirius* G5 i-antigen to capture recombinant G1 i-antigen produced in transformed *Tetrahymena.* Sera from test and control fish were added to wells and antibodies that bound to the captured i-antigen were detected using an alkaline phosphatase labeled mouse mAb against the immunoglobulin heavy chain of channel catfish. ELISA controls consisted of antibody-coated wells reacted with membrane protein from *Tetrahymena* cells transformed with the *neol* gene.

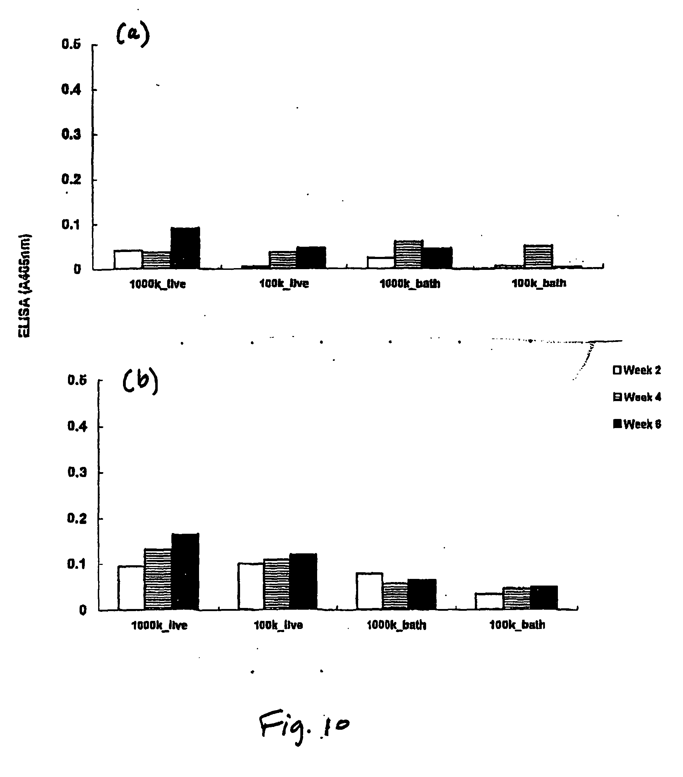

Fish injected with *Tetrahymena* membrane protein produced high levels of serum antibody against the recombinant i-antigen. The antibody response elicited by fish immunized with live cells was almost an order of magnitude lower. The antibody response of fish immunized by bath or i.p. injection with live cells secreting recombinant i-antigen was approximately two-fold greater than the antibody response of fish immunized with *Tetrahymena* producing the membrane-bound, intact i-antigen. In Fig. 10, the differences in antibody production between fish immunized with the (a) membrane associated or (b) secreted form of the i-antigen are shown. These results suggest that live cells secreting antigen are more efficacious in eliciting the production of serum antibodies. The mucosal antibody response was not determined in these experiments.

### Example VIII.

### Immune Response of Channel Catfish to Live Vaccine: Transformed Tetrahymena Expressing Full-length or Truncated I. multifiliis (Serotype A) 48 kD I-antigen Protein and Homologous Challenge with I. multifiliis (Serotype A)

*Tetrahymena* cells were transformed with either the entire *Ichthyophthirius* G1 48- kDa i-antigen protein, or a truncated gene sequence which encodes the i-antigen protein lacking 19 amino acids at the carboxy terminus as in Example VII.

Groups of channel catfish (70 fish per group) were vaccinated by intraperitoneal injection with 10⁶ *T. thermophila* transformants producing intact or truncated i-antigen. A third group of fish (control group) was vaccinated with *T. thermophila* transformants expressing *neo.* No adjuvant was used in any of the vaccinations. The fish were boosted 2 weeks following the initial injection and bled at 3 weeks following the initial injection. Sera from 3 fish per group were pooled.

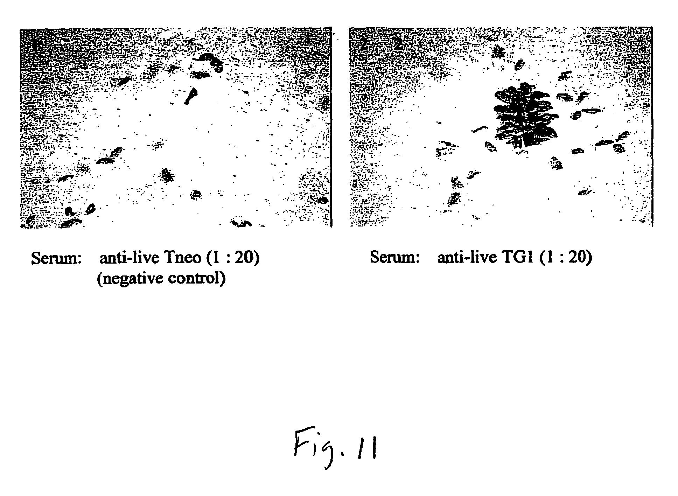

A 96 well ELISA plate was seeded with a homologous strain (i.e., serotype A) of *I. multifiliis* (strain NY1, a G1 isolate), 200 cells per well. Fish sera were serially diluted and added to the wells, and the effect on the motility of *I. multifliis* was observed. Immobilization of *I*. *multifiliis* was immediately evident at serum dilutions of 1:20, and at higher concentrations the organisms exhibited clumping (see Fig. 11). Sera from the control group did not cause any detectable change in motility of *I. multifiliis.*

As another control, additional wells were seeded with a heterologous strain of *I. multifiliis* (a G5 isolate). The motility of these organisms was not affected by sera from any of the groups of vaccinated fish, confirming that the immobilization epitopes on *I. multifiliis* i-antigens are highly specific.

For comparison, two other groups of fish were vaccinated with purified subunit proteins produced from recombinant *Tetrahymena* (either the full-length 48 kD i-antigen protein or the C-terminal truncated version). The subunit proteins were adjuvanted with Freund's Complete Adjuvant. In a plate assay similar to the one described above using the homologous strain of *I. multifiliis,* some immobilization was observed but not to the degree caused by the "live vaccine." This observation lends support to the expectation that the "live vaccine" will prove to be more efficacious than the analogous protein subunit vaccine.

## Claims

1. A recombinant protein expression system for heterologous proteins comprising:
a population of mutant ciliated protozoan host cells comprising a β-tubulin allele that confers paclitaxel sensitivity on the cells; and
a population of selectable transgenic ciliated protozoan host cells comprising a genomically integrated heterologous nucleic acid encoding a polypeptide, wherein the genomically integrated heterologous nucleic acid disrupts the β-tubulin allele thereby restoring paclitaxel resistance to the selectable cells such that the selectable cells are selectable by selection using paclitaxel; and
wherein the expressed heterologous protein is displayed on the plasma membrane surface of said transgenic protozoan host cell.

2. The recombinant protein expression system of claim 1 wherein the heterologous nucleic acid is derived from an organism that has an AT-rich genome.

3. The recombinant protein expression system of claim 1 or 2 wherein the protozoan host cell is a *Tetrahymena* host cell comprising, prior to genomic integration of the heterologous nucleic acid, a *btu1-1 K350M* β-tubulin allele.

4. A recombinant protein expression system comprising a transgenic ciliated protozoan host cell comprising a heterologous protein displayed on the plasma membrane surface of the host cell.

5. The recombinant protein expression system of claim 4 wherein the transgenic ciliated protozoan host cell is a *Tetrahymena* host cell.

6. The recombinant protein expression system of claim 4 or 5 wherein the surface-displayed heterologous protein comprises a GPI anchor encoded by a portion of an i-antigen-encoding nucleotide sequence from *I. multifiliis.*

7. A transgenic *T. thermophila* host cell comprising a heterologous protein displayed on the plasma membrane surface, wherein said heterologous protein comprises at least an antigenic portion of an *I. multifiliis* i-antigen protein.

8. The transgenic *T. thermophila* of claim 7 wherein the antigenic portion of the *I. multifiliis* i-antigen protein comprises a targeting amino acid sequence.

9. The transgenic *T. thermophila* of claim 8 wherein the targeting amino acid sequence comprises at least one of an N-terminal targeting sequence and a GPI cleavage/attachment sequence.

10. The transgenic *T. thermophila* of any of claims 7 to 9 comprising an *I. multifiliis* antigen protein displayed on the surface of its plasma membrane.

11. A transgenic cell comprising a heterologous protein displayed on the plasma membrane surface comprising at least one targeting amino acid sequence encoded by an i-antigen-encoding nucleotide sequence from *I. multifiliis.*

12. The transgenic cell of claim 11 wherein the targeting amino acid sequence comprises at least one of an N-terminal targeting sequence and a GPI cleavage/attachment sequence.

13. A method for making a polyclonal antibody comprising:
expressing a heterologous antigenic polypeptide on the surface of the plasma membrane of a transgenic protozoan host cell;
administering the transgenic protozoan host cell to a non-human animal to generate an antibody response to said antigenic polypeptide; and
isolating the antibody from the animal.

14. A method for making a polyclonal antibody comprising:
expressing a heterologous antigenic polypeptide on the surface of the plasma membrane of a transgenic protozoan host cell;
cleaving said antigenic polypeptide from the surface of the host cell;
isolating the antigenic polypeptide;
administering said antigenic polypeptide to a non-human animal to generate an antibody response to said antigenic polypeptide; and
isolating the antibody from the animal.

15. A method for detecting antibodies to a heterologous antigenic polypeptide comprising:
expressing said antigenic polypeptide on the surface of a transgenic protozoan host cell;
exposing the protozoan host cell to an antibody; and
determining whether the swimming behavior of the protozoan host cell is altered;
wherein alteration of the swimming behavior of the protozoan host cell is indicative of the presence of antibodies to said antigenic polypeptide.

16. The method of claim 15 wherein exposure to the antibody immobilizes the host cell.

17. The method of claim 15 or 16 wherein said heterologous antigenic polypeptide is a pathogenic parasite polypeptide, and wherein the host cell is exposed to the bodily fluid of a patient suspected of being infected with the parasite.

18. A method for screening drugs for the ability to bind a polypeptide comprising:
expressing a heterologous polypeptide on the surface of a transgenic protozoan host cell;
exposing the host cell to a drug; and
determining whether the swimming behavior of host cell is altered;
wherein an alteration in the swimming behavior of the host cell is indicative of binding of the drug to said polypeptide.

19. The method of claim 18 wherein the exposure to the drug immobilizes the host cell.

20. The method of claim 18 or 19 wherein said heterologous polypeptide is a human polypeptide.

21. A vaccine comprising a transgenic nonpathogenic immunogenic protozoan comprising a surface-displayed heterologous antigenic polypeptide.

22. The vaccine of claim 21 comprising a live vaccine.

23. The vaccine of claim 21 comprising a killed vaccine.

24. The vaccine of any of claims 21 to 23 wherein the transgenic nonpathogenic immunogenic protozoan is a transgenic nonpathogenic immunogenic ciliated protozoan.

25. The vaccine of claim 24 wherein the transgenic nonpathogenic immunogenic ciliated protozoan is *Tetrahymena.*

26. The vaccine of claim 25 wherein the *Tetrahymena* comprises a *btu1-1K350M* locus into which a heterologous nucleic acid encoding the antigenic polypeptide has been inserted.

27. The vaccine of any of claims 21 to 26 wherein said heterologous antigenic polypeptide comprises at least an antigenic portion of an *I. multifiliis* i-antigen protein.

28. Use of a transgenic nonpathogenic immunogenic protozoan comprising a surface-displayed heterologous antigenic polypeptide for the preparation of an immunostimulant in a vertebrate.

29. The use of claim 28 wherein the transgenic nonpathogenic immunogenic protozoan is a *Tetrahymena.*

30. The use of claim 28 or 29 wherein said heterologous antigenic polypeptide comprises at least an antigenic portion of an *l. multifiliis* i-antigen protein.

31. A recombinant method for producing a heterologous polypeptide comprising:
(a) providing a population of mutant protozoan host cells comprising a β-tubulin allele that confers paclitaxel sensitivity on the cells;
(b) introducing a heterologous nucleic acid encoding the polypeptide into the protozoan host cell such that it integrates into the genome of the protozoan host cells by disrupting the β-tubulin allele to yield a population of selectable transgenic protozoan host cells selectable by selection using paclitaxel; and
(c) expressing the polypeptide in the selectable transgenic protozoan host cells;
wherein said heterologous protein is displayed on the plasma membrane surface of the host cell.

32. The recombinant method of claim 31 wherein the protozoan host cell provided in step (a) is *Tetrahymena.*

33. The recombinant method of claim 32 wherein the *Tetrahymena* comprises a *btu1-1K350M β*-tubulin allele.

34. The recombinant method of any one of claims 31 to 33 further comprising:
(d) cleaving the polypeptide from the plasma membrane of the transgenic protozoan host cell.

35. The recombinant method of any of claims 31 to 34 wherein the polypeptide comprises at least one targeting amino acid sequence encoded by an i-antigen-encoding nucleotide sequence from *I. multifiliis.*

36. The recombinant method of any of claims 31 to 35 wherein the polypeptide is an antigenic polypeptide.

37. The recombinant method of claims 31 to 36 further comprising the step of:
isolating the polypeptide from the transgenic protozoan host cell.

38. A recombinant method for producing a heterologous polypeptide comprising:
(a) providing a ciliated protozoan host cell;
(b) introducing a heterologous nucleic acid encoding the polypeptide into the protozoan host cell to yield a transgenic ciliated protozoan host cell; and
(c) expressing the polypeptide in the transgenic ciliated protozoan host cell such that it is displayed on the plasma membrane surface of the host cell.

39. The recombinant method of claim 38 wherein said heterologous polypeptide comprises at least one targeting amino acid sequence encoded by an i-antigen-encoding nucleotide sequence from *I. multifiliis.*

40. The recombinant method of claim 38 or 39 further comprising:
(d) cleaving the polypeptide from the plasma membrane surface of the transgenic host cell.

41. The recombinant method of any of claims 38 to 40 wherein said heterologous polypeptide is an antigenic polypeptide.

42. The recombinant method of any of claims 38 to 41 wherein the transgenic ciliated protozoan host cell is *Tetrahymena.*

43. The recombinant protein expression system of any of claims 1 to 3 wherein the heterologous nucleic acid encodes a vertebrate polypeptide.

44. The recombinant protein expression system of claim 43 wherein the vertebrate polypeptide comprises a human polypeptide.

45. The recombinant protein expression system of any of claims 4 to 6 or the transgenic cell of claims 11 or 12 wherein the heterologous protein displayed on the plasma membrane surface of the host cell comprises a vertebrate polypeptide.

46. The recombinant protein expression system or the transgenic cell of claim 45 wherein the vertebrate polypeptide comprises a human polypeptide.

47. The method of any of claims 13 to 16, the vaccine of any of claims 21 to 26 or the use of claims 28 or 29 wherein the antigenic polypeptide comprises a vertebrate polypeptide.

48. The method, vaccine or use of claim 47 wherein the vertebrate polypeptide comprises a human polypeptide.

49. The recombinant method for producing a polypeptide of any of claims 31 to 42 wherein the heterologous nucleic acid encodes a vertebrate polypeptide.

50. The recombinant method for producing a polypeptide of any of claims 31 to 42 wherein the vertebrate polypeptide comprises a human polypeptide.

## Patentansprüche

1. Rekombinantes Protein-Expressionssystem für heterologe Proteine, das umfasst:
eine Population von mutierten bewimpertes Protozoon-Wirtszellen, die ein β-Tubulin-Allel umfassen, das Paclitaxel-Empfindlichkeit auf die Zellen überträgt; und
eine Population von auswählbaren transgenen bewimpertes Protozoon-Wirtszellen, die eine genomisch integrierte heterologe Nucleinsäure, die ein Polypeptid codiert, umfassen, wobei die genomischintegrierte heterologe Nucleinsäure das β-Tubulin-Allel unterbricht und dabei Paclitaxel-Resistenz der auswählbaren Zellen herstellt, so dass die auswählbaren Zellen durch Selektion unter Verwendung von Paclitaxel auswählbar sind; und
wobei das exprimierte heterologe Protein auf der Plasmamembranoberfläche der transgenen Protozoon-Wirtszelle gezeigt ist.

2. Rekombinantes Protein-Expressionssystem gemäß Anspruch 1, wobei die heterologe Nucleinsäure aus einem Organismus abgeleitet wird, der ein ATreiches Genom besitzt.

3. Rekombinantes Protein-Expressionssystem gemäß Anspruch 1 oder 2, wobei die Protozoon-Wirtszelle eine *Tetrahymena-Wirtszelle* ist, die, vor der genomischen Integration der heterologen Nucleinsäure, ein *btu-1-K350M*-β-Tubulin-Allel umfasst.

4. Rekombinantes Protein-Expressionssystem, das eine transgene bewimpertes Protozoon-Wirtszelle umfasst, die ein heterologes Protein umfasst, das auf der Plasmamembranoberfläche der Wirtszelle gezeigt ist.

5. Rekombinantes Protein-Expressionssystem gemäß Anspruch 4, wobei die transgene bewimpertes Protozoon-Wirtszelle eine *Tetrahymena-Wirtszelle* ist.

6. Rekombinantes Protein-Expressionssystem gemäß Anspruch 4 oder 5, wobei das auf der Oberfläche gezeigte heterologe Protein einen GPI-Anker umfasst, der durch einen Teil einer i-Antigen-codierenden Nucleotidsequenz von *I. multifiliis* codiert wird.

7. Transgene *T. thermophila*-Wirtszelle, die ein heterologes Protein umfasst, das auf der Plasmamembranoberfläche gezeigt ist, wobei das heterologe Protein mindestens einen antigenen Teil eines *I. multifiliis*-i-Antigen-Proteins umfasst.

8. Transgene *T. thermophila* gemäß Anspruch 7, wobei der antigene Teil des *I. multifiliis*-i-Antigen-Proteins eine zielgerichtete Aminosäuresequenz umfasst.

9. Transgene *T. thermophila* gemäß Anspruch 8, wobei die zielgerichtete Aminosäuresequenz mindestens eines von einer N-terminalen zielgerichteten Sequenz und einer GPI-Spaltung/Anlagerung-Sequenz umfasst.

10. Transgene *T. thermophila* gemäß einem der Ansprüche 7 bis 9, das ein *I.* multifilüs-Antigen-Protein umfasst, das auf der Oberfläche seiner Plasmamembran gezeigt ist.

11. Transgene Zelle, die ein heterologes Protein umfasst, das auf der Plasmamembranoberfläche gezeigt ist, das mindestens eine zielgerichtete Aminosäuresequenz umfasst, die durch eine i-Antigen-codierende Nucleotidsequenz von *I. multifiliis* codiert wird.

12. Transgene Zelle gemäß Anspruch 11, wobei die zielgerichtete Aminosäuresequenz mindestens eines von einer N-terminalen zielgerichteten Sequenz und einer GPI-Spaltung/Anlagerung-Sequenz umfasst.

13. Verfahren zum Herstellen eines polyclonalen Antikörpers, das umfasst:
das Exprimieren eines heterologen antigenen Polypeptids auf der Oberfläche der Plasmamembran einer transgenen Protozoon-Wirtszelle;
das Verabreichen der transgenen Protozoon-Wirtszelle an ein nicht-menschliches Tier, um eine Antikörperantwort auf das antigene Polypeptid zu erzeugen; und
das Isolieren des Antikörpers von dem Tier.

14. Verfahren zum Herstellen eines polyclonalen Antikörpers, das umfasst:
das Exprimieren eines heterologen antigenen Polypeptids auf der Oberfläche der Plasmamembran der transgenen Protozoon-Wirtszelle;
das Abspalten des antigenen Polypeptids von der Oberfläche der Wirtszelle;
das Isolieren des antigenen Polypeptids;
das Verabreichen des antigenen Polypeptids an ein nicht-menschliches Tier, um eine Antikörperantwort auf das antigene Polypepid zu erzeugen; und
das Isolieren des Antikörpers aus dem Tier.

15. Verfahren zum Nachweisen von Antikörpern gegen ein heterologes antigenes Polypeptid, das umfasst:
das Exprimieren des antigenen Polypeptids auf der Oberfläche einer transgenen Protozoon-Wirtszelle;
das Aussetzen der Protozoon-Wirtszelle einem Antikörper; und
das Ermitteln, ob das Schwimmverhalten der Protozoon-Wirtszelle verändert ist;
wobei eine Veränderung des Schwimmverhaltens der Protozoon-Wirtszelle die Anwesenheit von Antikörpern gegen das antigene Polypeptid anzeigt.

16. Verfahren gemäß Anspruch 15, wobei das Aussetzen gegenüber dem Antikörper die Wirtszelle immobilisiert.

17. Verfahren gemäß Anspruch 15 oder 16, wobei das heterologe antigene Polypeptid ein pathogener Parasit-Polypeptid ist, und wobei die Wirtszelle der Körperflüssigkeit eines Patienten ausgesetzt wird, bei dem der Verdacht besteht, mit dem Parasit infiziert zu sein.

18. Verfahren zum Durchmustern von Arzneistoffen auf die Fähigkeit, ein Polypeptid zu binden, das umfasst:
das Exprimieren eines heterologen Polypeptids auf der Oberfläche einer transgenen Protozoon-Wirtszelle;
das Aussetzen der Wirtszelle einem Arzneistoff; und
das Ermitteln, ob das Schwimmverhalten der Wirtszelle verändert ist;
wobei eine Veränderung im Schwimmverhalten der Wirtszelle die Bindung des Arzneistoffs an das Polypeptid anzeigt.

19. Verfahren gemäß Anspruch 18, wobei das Aussetzen gegenüber dem Arzneistoff die Wirtszelle immobilisiert.

20. Verfahren gemäß Anspruch 18 oder 19, wobei das heterologe Polypeptid ein menschliches Polypeptid ist.

21. Impfstoff, der ein transgenes nicht-pathogenes immunogenes Protozoon umfasst, das ein auf der Oberfläche gezeigtes heterologes antigenes Polypeptid umfasst.

22. Impfstoff gemäß Anspruch 21, der einen Lebendimpfstoff umfasst.

23. Impfstoff gemäß Anspruch 21, der einen abgetöteten Impfstoff umfasst.

24. Impfstoff gemäß einem der Ansprüche 21 bis 23, wobei das transgene nicht-pathogene immunogene Protozoon ein transgenes nicht-pathogenes immunogenes bewimpertes Protozoon ist.

25. Impfstoff gemäß Anspruch 24, wobei das transgene nicht-pathogene immunogene bewimperte Protozoon *Tetrahymena* ist.

26. Impfstoff gemäß Anspruch 25, wobei die *Tetrahymena* einen *btu1-1K350M-*Locus umfasst, in den eine heterologe Nucleinsäure eingebaut wurde, die das antigene Polypeptid codiert.

27. Impfstoff gemäß einem der Ansprüche 21 bis 26, wobei das heterologe antigene Polypeptid mindestens einen antigenen Teil eines *I. multifiliis*-i-Antigen-Proteins umfasst.

28. Verwendung eines transgenen nicht-pathogenen immunogenen Protozoons, das ein auf der Oberfläche gezeigtes heterologes antigenes Polypeptid umfasst, für die Herstellung eines Immunstimulans in einem Wirbeltier.

29. Verwendung gemäß Anspruch 28, wobei das transgene nicht-pathogene immunogene Protozoon eine *Tetrahymena* ist.

30. Verwendung gemäß Anspruch 28 oder 29, wobei das heterologe antigene Polypeptid mindestens einen antigenen Teil eines *I. multifiliis*-i-Antigen-Proteins umfasst.

31. Rekombinantes Verfahren zum Herstellen eines heterologen Polypeptids, das umfasst:
(a) das Bereitstellen einer Population von mutierten Protozoon-Wirtszellen, die ein β-Tubulin-Allel umfassen, das Paclitaxel-Empfindlichkeit auf die Zellen überträgt;
(b) das Einführen einer heterologen Nucleinsäure, die das Polypeptid codiert, in die Protozoon-Wirtszelle, so dass sie in das Genom der Protozoon-Wirtszelle integriert, wobei sie das β-Tubulin-Allel unterbricht, um eine Population von auswählbaren transgenen Protozoon-Wirtszellen hervorzubringen, die durch Selektion unter Verwendung von Paclitaxel auswählbar sind; und
(c) das Exprimieren des Polypeptids in den auswählbaren transgenen Protozoon-Wirtszellen;
wobei das heterologe Protein auf der Plasmamembranoberfläche der Wirtszelle gezeigt ist.

32. Rekombinantes Verfahren gemäß Anspruch 31, wobei die Protozoon-Wirtszelle, die in Schritt (a) bereitgestellt wird, *Tetrahymena* ist.

33. Rekombinantes Verfahren gemäß Anspruch 32, wobei die Tetrahymena ein *btu1-1K350M*-β-Tubulin-Allel umfasst.

34. Rekombinantes Verfahren gemäß einem der Ansprüche 31 bis 33, das ferner umfasst:
(d) das Abspalten des Polypeptids von der Plasmamembran der transgenen Protozoon-Wirtszelle.

35. Rekombinantes Verfahren gemäß einem der Ansprüche 31 bis 34, wobei das Polypeptid mindestens eine zielgerichtete Aminosäuresequenz umfasst, die durch eine i-Antigen-codierende Nucleotidsequenz von *I. multifiliis* codiert wird.

36. Rekombinantes Verfahren gemäß einem der Ansprüche 31 bis 35, wobei das Polypeptid ein antigenes Polypeptid ist.

37. Rekombinantes Verfahren gemäß einem der Ansprüche 31 bis 36, das ferner den Schritt umfasst:
das Isolieren des Polypeptids aus der transgenen Protozoon-Wirtszelle.

38. Rekombinantes Verfahren zum Herstellen eines heterologen Polypeptids, das umfasst:
(a) das Bereitstellen einer bewimpertes Protozoon-Wirtszelle;
(b) das Einführen einer heterologen Nucleinsäure, die das Polypeptid codiert, in die Protozoon-Wirtszelle, um eine transgene bewimpertes Protozoon-Wirtszelle hervorzubringen; und
(c) das Exprimieren des Polypeptids in der transgenen bewimpertes Protozoon-Wirtszelle, so dass es auf der Plasmamembranoberfläche der Wirtszelle gezeigt ist.

39. Rekombinantes Verfahren gemäß Anspruch 38, wobei das heterologe Polypeptid mindestens eine zielgerichtete Aminosäuresequenz umfasst, die durch eine i-Antigen-codierende Nucleotidsequenz von *I. multifiliis* codiert wird.

40. Rekombinantes Verfahren gemäß Anspruch 38 oder 39, das ferner umfasst:
(d) das Abspalten des Polypeptids von der Plasmamembranoberfläche der transgenen Wirtszelle.

41. Rekombinantes Verfahren gemäß einem der Ansprüche 38 bis 40, wobei das heterologe Polypeptid ein antigenes Polypeptid ist.

42. Rekombinantes Verfahren gemäß einem der Ansprüche 38 bis 41, wobei die transgene bewimpertes Protozoon-Wirtszelle Tetrahymena ist.

43. Rekombinantes Protein-Expressionssystem gemäß einem der Ansprüche 1 bis 3, wobei die heterologe Nucleinsäure ein Wirbeltier-Polypeptid codiert.

44. Rekombinantes Protein-Expressionssystem gemäß Anspruch 43, wobei das Wirbeltier-Polypeptid ein menschliches Polypeptid umfasst.

45. Rekombinantes Protein-Expressionssystem gemäß einem der Ansprüche 4 bis 6, oder transgene Zelle gemäß Anspruch 11 oder 12, wobei das heterologe Protein, das auf der Plasmamembranoberfläche der Wirtszelle gezeigt ist, ein Wirbeltier-Polypeptid umfasst.

46. Rekombinantes Protein-Expressionssystem oder transgene Zelle gemäß Anspruch 45, wobei das Wirbeltier-Polypeptid ein menschliches Polypeptid umfasst.

47. Verfahren gemäß einem der Ansprüche 13 bis 16, Impfstoff gemäß einem der Ansprüche 21 bis 26 oder Verwendung gemäß Anspruch 28 oder 29, wobei das antigene Polypeptid ein Wirbeltier-Polypeptid umfasst.

48. Verfahren, Impfstoff oder Verwendung gemäß Anspruch 47, wobei das Wirbeltier-Polypeptid ein menschliches Polypeptid umfasst.

49. Rekombinantes Verfahren zum Herstellen eines Polypeptids gemäß einem der Ansprüche 31 bis 42, wobei die heterologe Nucleinsäure ein Wirbeltier-Polypeptid codiert.

50. Rekombinantes Verfahren zum Herstellen eines Polypeptids gemäß einem der Ansprüche 31 bis 42, wobei das Wirbeltier-Polypeptid ein menschliches Polypeptid umfasst.

## Revendications

1. Système d'expression de protéines recombinantes pour des protéines hétérologues comprenant :
une population de cellules hôtes de protozoaire cilié mutant comprenant un allèle de β-tubuline qui confère aux cellules une sensibilité au paclitaxel ; et
une population de cellules hôtes de protozoaire cilié transgénique comprenant un acide nucléique hétérologue intégré au niveau du génome codant pour un polypeptide, où l'acide nucléique hétérologue intégré au niveau du génome interrompt l'allèle de β-tubuline, restaurant ainsi la résistance au paclitaxel dans les cellules sélectionnables de telle manière que les cellules sélectionnables sont sélectionnables en utilisant du paclitaxel ; et
où la protéine hétérologue exprimée est présentée à la surface de la membrane plasmatique de ladite cellule hôte de protozoaire transgénique.

2. Système d'expression de protéines recombinantes selon la revendication 1, dans lequel l'acide nucléique hétérologue est dérivé d'un organisme qui a un génome riche en AT.

3. Système d'expression de protéines recombinantes selon la revendication 1 ou 2, dans lequel la cellule hôte de protozoaire est une cellule hôte de *Tetrahymena* comprenant, avant l'intégration génomique de l'acide nucléique hétérologue, un allèle de β-tubuline *btu1-1 K350M.*

4. Système d'expression de protéines recombinantes comprenant une cellule hôte de protozoaire cilié transgénique comprenant une protéine hétérologue présentée à la surface de la membrane plasmatique de la cellule hôte.

5. Système d'expression de protéines recombinantes selon la revendication 4, dans lequel la cellule hôte de protozoaire cilié transgénique est une cellule hôte de *Tetrahymena.*

6. Système d'expression de protéines recombinantes selon la revendication 4 ou 5, dans lequel la protéine hétérologue présentée à la surface comprend une ancre GPI codée par une partie d'une séquence nucléotidique codant pour l'antigène i de *I. multifiliis.*

7. Cellule hôte de *T. thermophila* transgénique comprenant une protéine hétérologue présentée à la surface de la membrane plasmatique, où ladite protéine hétérologue comprend au moins une partie antigénique de la protéine de l'antigène i de *I. multifiliis.*

8. *T. thermophila* transgénique selon la revendication 7, où la partie antigénique de la protéine de l'antigène i de *I. multifiliis* comprend une séquence d'acides aminés ciblante.

9. *T. thermophila* transgénique selon la revendication 8, où la séquence d'acides aminés ciblante comprend au moins une séquence ciblante N-terminale et une séquence de clivage/fixation de GPI.

10. *T. thermophila* transgénique selon l'une quelconque des revendications 7 à 9 comprenant une protéine d'antigène de *I. multifiliis* présentée à la surface de sa membrane plasmatique.

11. Cellule transgénique comprenant une protéine hétérologue présentée à la surface de la membrane plasmatique comprenant au moins une séquence d'acides aminés ciblante codée par une séquence nucléotidique codant pour l'antigène i de *I multifiliis.*

12. Cellule transgénique selon la revendication 11, où la séquence d'acides aminés ciblante comprend au moins une séquence ciblante N-terminale et une séquence de clivage/fixation de GPI.

13. Procédé de fabrication d'un anticorps polyclonal comprenant :
l'expression d'un polypeptide antigénique hétérologue à la surface de la membrane plasmatique d'une cellule hôte de protozoaire transgénique ;
l'administration de la cellule hôte de protozoaire transgénique à un animal non humain pour générer une réponse en anticorps au dit polypeptide antigénique ; et
l'isolement de l'anticorps à partir de l'animal.

14. Procédé de fabrication d' un anticorps polyclonal comprenant :
l'expression d'un polypeptide antigénique hétérologue à la surface de la membrane plasmatique d'une cellule hôte de protozoaire transgénique ;
le clivage dudit polypeptide antigénique de la surface de la cellule hôte ;
l'isolement du polypeptide antigénique ;
l'administration dudit polypeptide antigénique à un animal non humain pour générer une réponse en anticorps au dit polypeptide antigénique ; et
l'isolement de l'anticorps à partir de l'animal.

15. Procédé de détection des anticorps dirigés contre un polypeptide antigénique hétérologue comprenant :
l'expression dudit polypeptide antigénique à la surface d'une cellule hôte de protozoaire transgénique ;
l'exposition de la cellule hôte de protozoaire à un anticorps ; et
la détermination de l'existence d'une modification du comportement natatoire de la cellule hôte de protozoaire;
où une modification du comportement natatoire de la cellule hôte de protozoaire est indicatrice de la présence d'anticorps dirigés contre ledit polypeptide antigénique.

16. Procédé selon la revendication 15, dans lequel l'exposition à l'anticorps immobilise la cellule hôte.

17. Procédé selon la revendication 15 ou 16, dans lequel ledit polypeptide antigénique hétérologue est un polypeptide de parasite pathogène, et dans lequel la cellule hôte est exposée au liquide corporel d'un patient suspecté d'être infecté par le parasite.

18. Procédé de criblage de médicaments pour leur capacité de se lier à un polypeptide comprenant :
l'expression d'un polypeptide hétérologue à la surface d'une cellule hôte de protozoaire transgénique ;
l'expression de la cellule hôte à un médicament ; et
la détermination de l'existence d'une modification du comportement natatoire de la cellule hôte ;
où une modification du comportement natatoire de la cellule hôte est indicatrice de la liaison du médicament au dit polypeptide.

19. Procédé selon la revendication 18, dans lequel l'exposition au médicament immobilise la cellule hôte.

20. Procédé selon la revendication 18 ou 19, dans lequel ledit polypeptide hétérologue est un polypeptide humain.

21. Vaccin comprenant un protozoaire immunogénique non pathogène transgénique comprenant un polypeptide antigénique hétérologue présenté à la surface.

22. Vaccin selon la revendication 21 comprenant un vaccin vivant.

23. Vaccin selon la revendication 21 comprenant un vaccin tué.

24. Vaccin selon l'une quelconque des revendications 21 à 23, dans lequel le protozoaire immunogénique non pathogène transgénique est un protozoaire cilié immunogénique non pathogène transgénique.

25. Vaccin selon la revendication 24, dans lequel le protozoaire cilié immunogénique non pathogène transgénique est *Tetrahymena.*

26. Vaccin selon la revendication 25, dans lequel le *Tetrahymena* comprend un locus *btu1-1K350M* dans lequel un acide nucléique hétérologue codant pour le polypeptide antigénique a été inséré.

27. Vaccin selon l'une quelconque des revendications 21 à 26, dans lequel ledit polypeptide antigénique hétérologue comprend au moins une partie antigénique d'une protéine de l'antigène i de *I. multifiliis.*

28. Utilisation d'un protozoaire immunogénique non pathogène transgénique comprenant un polypeptide antigénique hétérologue présenté à la surface pour la préparation d'un immunostimulant chez un vertébré.

29. Utilisation selon la revendication 28, où le protozoaire immunogénique non pathogène transgénique est un *Tetrahymena.*

30. Utilisation selon la revendication 28 ou 29, où ledit polypeptide antigénique hétérologue comprend au moins une partie antigénique d'une protéine de l'antigène i de *I. multifiliis.*

31. Procédé recombinant de production d'un polypeptide hétérologue comprenant :
(a) la fourniture d'une population de cellules hôtes de protozoaire mutant comprenant un allèle de β-tubuline qui confère aux cellules une sensibilité au paclitaxel ;
(b) l'introduction d'un acide nucléique hétérologue codant pour le polypeptide dans la cellule hôte de protozoaire de telle manière qu'il s'intègre dans le génome des cellules hôtes de protozoaire en interrompant l'allèle de β-tubuline pour produire une population de cellules hôtes sélectionnables de protozoaire transgénique par sélection en utilisant du paclitaxel ; et
(c) l'expression du polypeptide dans les cellules hôtes sélectionnables de protozoaire transgénique ;
où ladite protéine hétérologue est présentée à la surface de la membrane plasmatique de la cellule hôte.

32. Procédé recombinant selon la revendication 31, dans lequel la cellule hôte de protozoaire dans l'étape (a) est *Tetrahymena.*

33. Procédé recombinant selon la revendication 32, dans lequel le *Tetrahymena* comprend un allèle de β-tubuline *btu1-1K350M.*

34. Procédé recombinant selon l'une quelconque des revendications 31 à 33 comprenant en outre :
(d) le clivage du polypeptide de la membrane plasmatique de la cellule hôte de protozoaire transgénique.

35. Procédé recombinant selon l'une quelconque des revendications 31 à 34, dans lequel le polypeptide comprend au moins une séquence d'acides aminés ciblante codée par une séquence nucléotidique codant pour l'antigène i de *I. multifiliis.*

36. Procédé recombinant selon l'une quelconque des revendications 31 à 35, dans lequel le polypeptide est un polypeptide antigénique.

37. Procédé recombinant selon les revendications 31 à 36 comprenant en outre l'étape consistant à:
isoler le polypeptide à partir de la cellule hôte de protozoaire transgénique.

38. Procédé recombinant de production d'un polypeptide hétérologue comprenant :
(a) la fourniture d'une cellule hôte de protozoaire cilié ;
(b) l'introduction d'un acide nucléique hétérologue codant pour le polypeptide dans la cellule hôte de protozoaire pour produire une cellule hôte de protozoaire cilié transgénique ; et
(c) l'expression du polypeptide dans la cellule hôte de protozoaire cilié transgénique de telle manière qu'il est présenté à la surface de la membrane plasmatique de la cellule hôte.

39. Procédé recombinant selon la revendication 38, dans lequel ledit polypeptide hétérologue comprend au moins une séquence d'acides aminés ciblante codée par une séquence nucléotidique codant pour l'antigène i de *I. multifiliis.*

40. Procédé recombinant selon la revendication 38 ou 39 comprenant en outre :
(d) le clivage du polypeptide à partir de la surface de la membrane plasmatique de la cellule hôte transgénique.

41. Procédé recombinant selon l'une quelconque des revendications 38 à 40, dans lequel ledit polypeptide hétérologue est un polypeptide antigénique.

42. Procédé recombinant selon l'une quelconque des revendications 38 à 41, dans lequel la cellule hôte de protozoaire cilié transgénique est *Tetrahymena.*

43. Système d'expression de protéines recombinantes selon l'une quelconque des revendications 1 à 3, dans lequel l'acide nucléique hétérologue code pour un polypeptide de vertébré.

44. Système d'expression de protéines recombinantes selon la revendication 43, dans lequel le polypeptide de vertébré comprend un polypeptide humain.

45. Système d'expression de protéines recombinantes selon l'une quelconque des revendications 4 à 6 ou cellule transgénique selon la revendication 11 ou 12, où la protéine hétérologue présentée à la surface de la membrane plasmatique de la cellule hôte comprend un polypeptide de vertébré.

46. Système d'expression de protéines recombinantes ou cellule transgénique selon la revendication 45, où le polypeptide de vertébré comprend un polypeptide humain.

47. Procédé selon l'une quelconque des revendications 13 à 16, vaccin selon l'une quelconque des revendications 21 à 26 ou utilisation selon la revendication 28 ou 29, où le polypeptide antigénique comprend un polypeptide de vertébré.,

48. Procédé, vaccin ou utilisation selon la revendication 47, où le polypeptide de vertébré comprend un polypeptide humain.

49. Procédé recombinant de production d'un polypeptide selon l'une quelconque des revendications 31 à 42, dans lequel l'acide nucléique hétérologue code pour un polypeptide de vertébré.

50. Procédé recombinant de production d'un polypeptide selon l'une quelconque des revendications 31 à 42, dans lequel le polypeptide de vertébré comprend un polypeptide humain.
